# EUROPEAN PATENT SPECIFICATION

(11) **EP 4 491 080 B1**
(45) Date of publication and mention of the grant of the patent: **04.11.2026**
(21) Application number: 23767024.5
(22) Date of filing: 08.02.2023
(51) Int. Cl.: A47L 9/14, A47L 9/28, A47L 7/00, A47L 9/00, A47L 5/24, A61L 2/08

(54) **CLEANER STATION**
REINIGUNGSSTATION
STATION DE DISPOSITIF DE NETTOYAGE

(30) Priority: 10.03.2022 KR 20220030167
(43) Date of publication of application: 15.01.2025
(73) Proprietor: LG Electronics Inc., Yeongdeungpo-gu Seoul 07336 (KR)
(72) Inventor: LEE, Donggeun, Seoul 08592 (KR); RYU, Jungwan, Seoul 08592 (KR); KIM, Sungjun, Seoul 08592 (KR)
(74) Representative: Ter Meer und Partner mbB
(86) International application number: PCT/KR2023/001809
(87) International publication number: WO 2023/171919

(56) References cited:
- JP-A- 2017 189 453
- JP-A- H08 173 359
- JP-A- H08 206 044
- KR-A- 20220 027 649
- KR-A- 20220 027 649
- KR-B1- 102 161 708
- KR-B1- 102 161 708
- KR-B1- 102 354 485

## Description

### [Technical Field]

The present invention relates to a cleaner station for suctioning dust stored in a cleaner into an inside thereof, and more specifically, to a cleaner station in which a detachable structure of a dust bag for storing the suctioned dust is simple and stable.

### [Background Art]

In general, a cleaner is a home appliance for suctioning small trash or dust in a manner of suctioning air using electricity and filling the same in a dust bin inside a product and is commonly called a vacuum cleaner.

The vacuum cleaner may be classified into a manual vacuum cleaner for allowing a user to directly perform cleaning while moving the cleaner, and an automatic vacuum cleaner for performing cleaning while traveling by itself. Depending on the type of the vacuum cleaner, the manual vacuum cleaner may be classified into a canister-type vacuum cleaner, an upright vacuum cleaner, a hand vacuum cleaner, a stick-type vacuum cleaner, etc.

In the past, the canister-type vacuum cleaner was widely used as the household vacuum cleaner, but recently, the hand vacuum cleaner and the stick-type vacuum cleaner, which provide improved convenience of use by integrally providing a dust bin and a cleaner body, are increasingly being used.

The canister-type vacuum cleaner has a main body and a suction port connected by a rubber hose or a pipe and in some cases, may be used by inserting a brush into the suction port.

The hand vacuum cleaner is designed to maximize portability and has lightweight and a short length, and thus may have a limited cleaning area. Therefore, the hand vacuum cleaner is used to clean localized sites, such as on a desk, a sofa, or a vehicle interior.

A user may use the stick-type vacuum cleaner while standing to enable cleaning without bending down. Therefore, it is advantageous for cleaning a wide region while moving. While the hand vacuum cleaner cleans narrow spaces, the stick-type vacuum cleaner may clean wider spaces and clean high places out of reach. Recently, the stick-type vacuum cleaner has been provided in a module type to allow users to actively change a vacuum cleaner type for various purposes.

However, the stick-type vacuum cleaner has a small capacity of a dust bin for storing the collected dust, thereby causing the user's inconvenience having to empty the dust bin frequently. In general, a cleaner is a home appliance for suctioning small trash or dust in a manner of suctioning air using electricity and filling the same in a dust bin inside a product and is commonly called a vacuum cleaner.

The vacuum cleaner may be classified into a manual vacuum cleaner for allowing a user to directly perform cleaning while moving the cleaner, and an automatic vacuum cleaner for performing cleaning while traveling by itself. Depending on the type of the vacuum cleaner, the manual vacuum cleaner may be classified into a canister-type vacuum cleaner, an upright vacuum cleaner, a hand vacuum cleaner, a stick-type vacuum cleaner, etc.

In the past, the canister-type vacuum cleaner was widely used as the household vacuum cleaner, but recently, the hand vacuum cleaner and the stick-type vacuum cleaner, which provide improved convenience of use by providing a dust bin and a cleaner body, are increasingly being used.

The canister-type vacuum cleaner has a main body and a suction port connected by a rubber hose or a pipe and in some cases, may be used by inserting a brush into the suction port.

The hand vacuum cleaner is designed to maximize portability and has lightweight and a short length, and thus may have a limited cleaning area. Therefore, the hand vacuum cleaner is used to clean localized sites, such as on a desk, a sofa, or a vehicle interior.

A user may use the stick-type vacuum cleaner while standing to enable cleaning without bending down. Therefore, it is advantageous for cleaning a wide region while moving. While the hand vacuum cleaner cleans narrow spaces, the stick-type vacuum cleaner may clean wider spaces and clean high places out of reach. Recently, the stick-type vacuum cleaner has been provided in a module type to allow users to actively change a vacuum cleaner type for various purposes.

However, the stick-type vacuum cleaner has a small capacity of a dust bin for storing the collected dust, thereby causing the user's inconvenience having to empty the dust bin frequently.

As Patent Document 1, Korean Patent Application Laid-Open No. 2020-0074001 discloses a cleaning device including a vacuum cleaner and a docking station.

Patent Document 1 includes a vacuum cleaner including a dust collection bin for collecting a foreign substance, and a docking station connected to the dust collection bin to remove the foreign substance collected in the dust collection bin, in which the dust collection bin is provided to be docked to the docking station, and the docking station includes a suction device for suctioning the foreign substance and internal air in the docked dust collection bin.

In addition, the docking station of Patent Document 1 may further include a collecting unit for collecting a foreign substance, and the collecting unit is a component that may be separated from the docking station and when the foreign substance fully fills the collecting unit, a user may replace a dust bag disposed in the collecting unit.

In this case, when the user drives the suction device of the docking station in a state in which the collecting unit of Patent Document 1 is separated from the docking station and the dust bag is not yet replaced, there is a problem that the foreign substance of the vacuum cleaner flows into the docking station, causing a failure of the suction device. However, Patent Document 1 does not disclose a solution to such a problem.

In this regard, as Patent Document 2, Korean Patent No. 2161708 is presented.

A station disclosed in Patent Document 2 includes a housing in which a dust bag is disposed, a holder to which the dust bag is detachably attached and which rotates in the housing, and a cover for opening and closing one area of the housing.

Patent Document 2 is similar to Patent Document 1 in that it is configured to separate the dust bag by opening the cover, but differs from Patent Document 1 in that it further includes a lever formed to interfere with the cover in a state in which the dust bag is not inserted into the holder. According to Patent Document 2, in a state in which the dust bag is not inserted into the housing, the holder and the lever each rotate in a direction that interferes with the cover, and thus the cover cannot be closed. Therefore, there is an advantage in that the user structurally recognizes a state in which the dust bag is not installed.

However, in Patent Document 2, the lever should be provided separately from the holder for installing the dust bag, and a torsion spring configured to ensure that the lever continuously receives a force to rotate. That is, there are a problem of increased manufacturing costs and a problem of a complicated structure because a plurality of parts are required.

In addition, when a rotational structure is applied to the plurality of parts as in Patent Document 2, there is also a problem that a product is vulnerable to failure.

KR 102 161 708 B1 discloses a vacuum cleaner station according to the preamble of independent claims 1 and 14. The vacuum cleaner station comprises: a reception chamber docked with a dust box; a housing in which a dust bag is arranged; a cover opening/closing one area of the housing; a holder allowing the dust bag to be selectively attached/detached and allowing the dust bag to selectively communicate with the reception chamber; and a lever arranging one end in one area of the housing to rotate in a first direction to selectively interfere with the cover. When the dust bag is installed in the holder to communicate with the reception chamber, the lever rotates in a second direction opposite to the first direction to close the cover.

### [Invention]

### [Technical Problem]

The present invention is directed to providing a cleaner station with a simplified dust bag recognition structure and improved durability.

In addition, the present invention is directed to providing a cleaner station capable of easily recognizing whether a dust bag is in a state of being disposed at a correct location.

### [Technical Solution]

To achieve the objects, according to an embodiment of the present invention, there is provided a cleaner station connected to a dust bin of a cleaner to suction dust from the dust bin.

The cleaner station includes a dust storage part configured to store dust suctioned from the cleaner, a dust storage housing part in which an accommodation space having the dust storage part accommodated therein is formed and in which the dust storage part is detachably provided, and a housing cover part configured to open and close a partial area of the dust storage housing part.

The dust storage housing part includes a fixed holder fixedly disposed to an upper surface of the accommodation space and including an installation groove formed so that the dust storage part is formed to slide, and a stopper connected to the fixed holder to linearly reciprocate a predetermined distance.

The stopper linearly moves in a direction that interferes with the housing cover part when the dust storage part is separated from the installation groove.

The stopper may be linearly moved to a first location by being pushed while being supported by a partial area of the dust storage part when the dust storage part slides in a direction that is inserted into the installation groove.

The stopper may linearly move to a second location interfering with a partial area of the housing cover part as the support by the dust storage part is released when the dust storage part slides in a direction that is separated from the installation groove.

The linear movement from the first location to the second location may be a downward movement by gravity.

A spring configured to apply an elastic restoring force to the second location may be coupled to the stopper.

The linear movement from the first location to the second location may be a movement by the elastic restoring force of the spring.

The fixed holder may further includes a guide rail configured to guide the stopper to linearly move in a vertical direction.

The stopper may include a rail insertion groove into which the guide rail is inserted, and a hook protruding and extending from a rear end of the rail insertion groove to a center of the stopper and coupled by being caught on the guide rail.

The fixed holder may further include a rail groove formed from a front surface to a rear and providing a space in which the stopper moves in a vertical direction, and a support surface supporting a partial surface of the stopper at a lower end portion of the rail groove to stop the downward movement.

The fixed holder may further include a first installation surface disposed on a path along which the dust storage part slides and extending in a horizontal direction, and a second installation surface connected to a rear end of the first installation surface and extending to have a rearward and upward inclination.

The housing cover part may include an interference member protruding toward an inside of the dust storage housing part.

The interference member may be disposed at a location at which closing of the dust storage housing part is interfered by the stopper in a state in which the dust storage part has been separated from the installation groove.

The dust storage part may include a dust bag configured to accommodate and store dust suctioned from the cleaner therein, and an upper plate provided to be coupled to an external upper surface of the dust bag and inserted into the installation groove in a sliding manner.

The upper plate may include a dust bag handle provided at a front of the dust storage part and gripped by a user, and an inclined rib formed to protrude and extend rearward from both left and right end portions of the dust bag handle and formed to extend to have a rearward and upward inclination.

The stopper may include a guide inclined surface having a downward inclination in an insertion direction of the upper plate.

In an embodiment of the present invention, when the upper plate is inserted into the installation groove, the stopper may move upward as the upper plate slides toward an inside of the accommodation space along the guide inclined surface.

The dust storage part may further include a lower plate coupled to an inner upper surface of the dust bag and provided with a transmissive panel to transmit sterilization light.

The cleaner station may further include a micro switch in contact with the dust storage part to detect whether the dust storage part has been disposed at a correct location when the dust storage part is inserted into the dust storage housing part.

In an embodiment of the present invention for achieving the object, one sides of the installation groove and the dust storage part are each provided with a convex portion and the other sides are each provided with a concave portion having a shape corresponding to the convex portion.

In the embodiment of the present invention, when the dust storage part is disposed by being inserted at a correction location of the dust storage housing part, the convex portion and the concave portion are coupled.

The convex portion or the concave portion may be provided on one end portion of a sliding movement path of the upper plate.

### [Advantageous Effects]

According to the present invention, disclosed is the simplified dust bag recognition structure composed of only the stopper moving linearly. Therefore, there is an advantage of the cost reduction of parts in the cleaner station.

In addition, according to the present invention, since the stopper reciprocates linearly, there is an advantage of being resistant to failure and wear and having improved durability compared to when the rotating part is used.

In addition, according to the present invention, the user can easily recognize, through touch, the state in which the dust bag has been installed at the correct location of the cleaner station through the fastening structure composed of the convex portion and the concave portion.

The effects of the present invention are not limited to the above-described effects, and other effects that are not described will be able to be clearly understood by those skilled in the art from the above detailed description.

### [Description of Drawings]

FIG. 1 is a perspective view showing a dust removal system including a cleaner station and a cleaner according to the present invention.
FIG. 2 is a view showing a shape in which the cleaner is coupled to the cleaner station and an inside of a side surface of the cleaner station according to the present invention.
FIG. 3 is an enlarged view of a dust bin opening/closing structure of the cleaner according to the present invention.
FIG. 4 is an enlarged view of a cover opening unit according to the present invention.
FIG. 5 is an enlarged view of a door unit according to the present invention.
FIG. 6 is an exploded view of main components of a dust storage part according to the present invention.
FIG. 7 is an enlarged view of an upper plate of the dust storage part according to the present invention.
FIG. 8 is a view showing a state in which the dust storage part has been accommodated in a dust storage housing part according to the present invention.
FIG. 9 is an enlarged view of fixed holders according to the present invention.
FIG. 10 is a front view of the fixed holders according to the present invention.
FIG. 11 is a view showing a state in which a stopper coupled to the fixed holders is located at each of a first location and a second location according to the present invention.
FIG. 12 is a perspective view of the stopper when viewed from the rear according to the present invention.
FIG. 13 is a perspective view of the stopper when viewed from the front according to the present invention.
FIG. 14 is a perspective view separately showing main components to describe a coupling relationship between the dust storage part and the fixed holders according to the present invention.
FIG. 15 is a conceptual diagram for sequentially describing a process in which the dust storage part is inserted into the dust storage housing part and a movement path of the stopper accordingly according to the present invention.
FIG. 16 is a view for describing an embodiment in which a micro switch is provided according to the present invention.
FIG. 17 is a view for describing another embodiment in which a dust bag recognition structure is possible according to the present invention.
FIG. 18 is a view for describing a correction location installation detection structure of the dust bag according to the present invention.
FIG. 19 is a cross-sectional view showing a state in which the dust bag has been installed at the correct location in the embodiment of FIG. 18.

### [Mode for Invention]

Hereinafter, exemplary embodiments of the present invention will be described in detail with reference to the accompanying drawings.

Since the present invention may have various changes and various embodiments, specific embodiments will be shown in drawings and described in detail in detailed descriptions. This is not intended to limit the present invention to specific embodiments and technical scope of the present invention is defined only by the appended claims.

The terms used in the present application are only used to describe specific embodiments and are not intended to limit the present invention. The singular expression includes the plural expression unless the context clearly dictates otherwise.

Unless defined otherwise, all terms used herein, including technical or scientific terms, have the same meaning as commonly understood by those skilled in the art to which the present invention pertains. The terms defined in a generally used dictionary can be construed as meanings that match with the meanings of the terms from the context of the related technology and are not construed as an ideal or excessively formal meaning unless clearly defined in the present application.

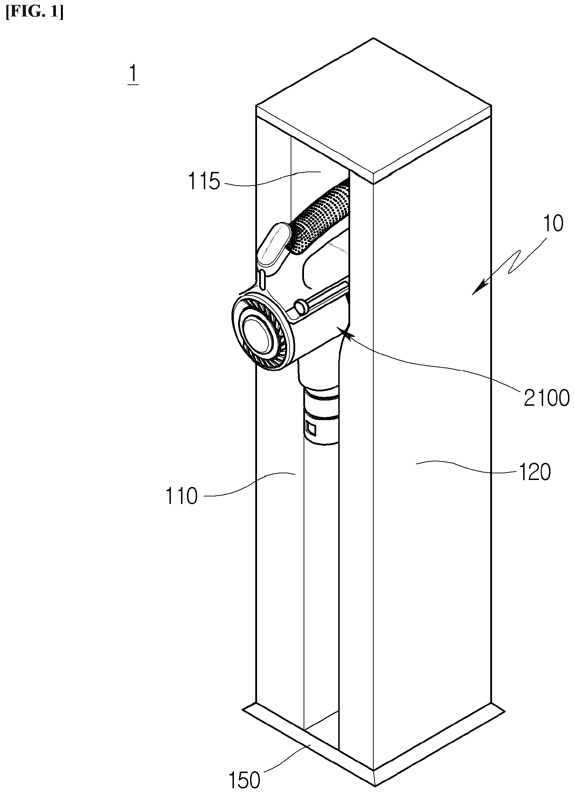

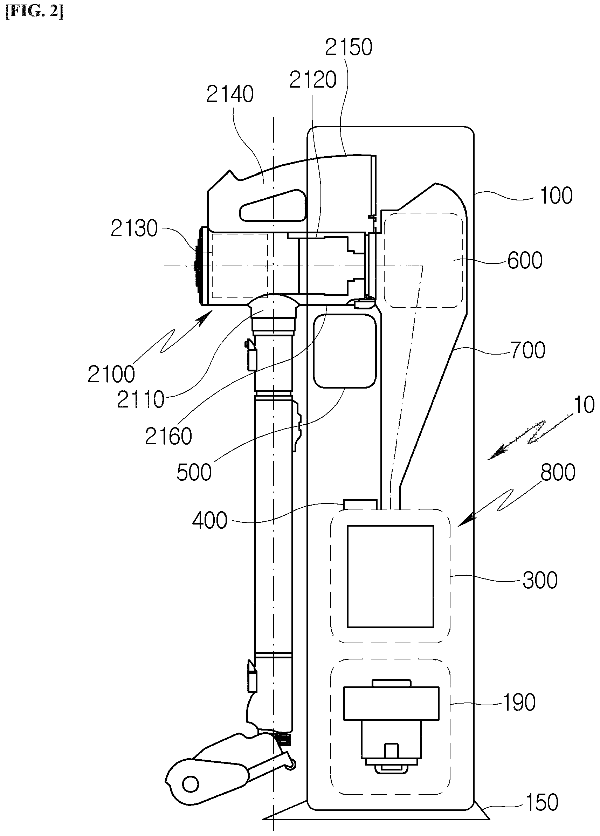

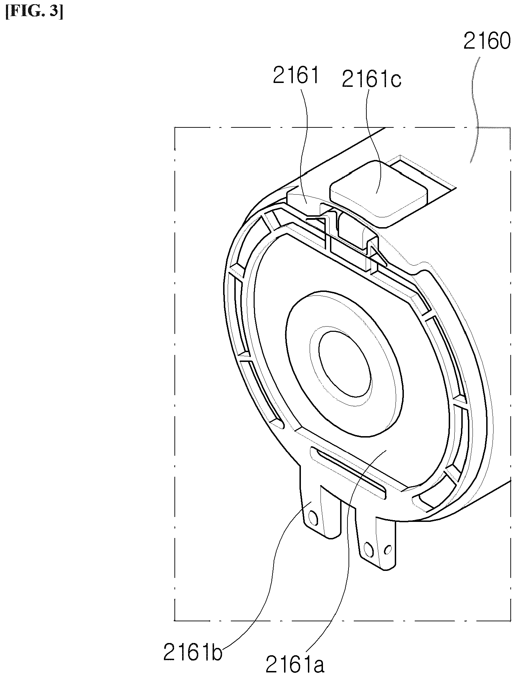

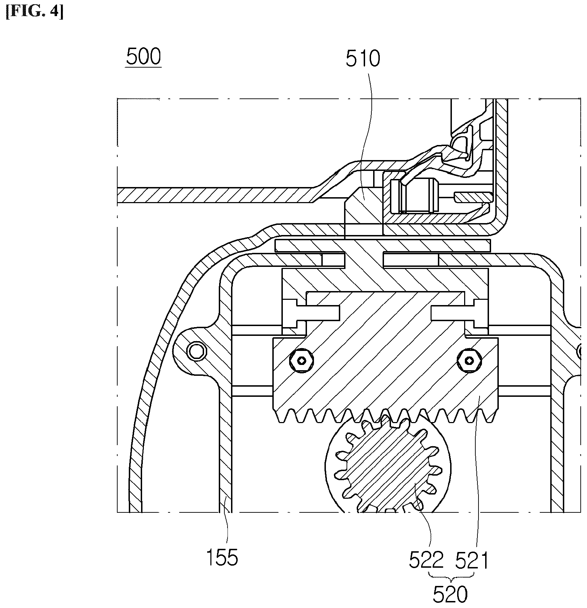

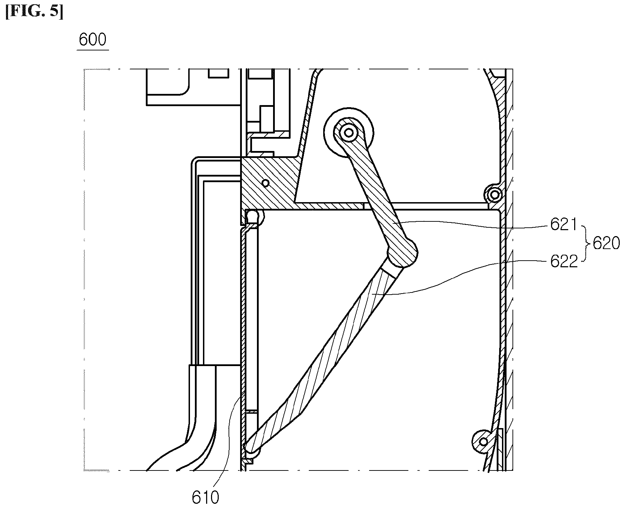

FIG. 1 is a perspective view showing a dust removal system including a cleaner station and a cleaner according to the present invention, FIG. 2 is a view showing a shape in which the cleaner is coupled to the cleaner station and an inside of a side surface of the cleaner station according to the present invention, FIG. 3 is an enlarged view of a dust bin opening/closing structure of the cleaner according to the present invention, FIG. 4 is an enlarged view of a cover opening unit according to the present invention, and FIG. 5 is an enlarged view of a door unit according to the present invention.

Referring to FIGS. 1 to 5, a dust removal system 1 may include a cleaner station 10 and a cleaner 20.

The cleaner 20 may be coupled to a front of the cleaner station 10. More specifically, a cleaner body 2100 of the cleaner 20 may be coupled to the front of the cleaner station 10.

In this case, the front of the cleaner station 10 can be defined as a direction in which a coupling part 115, which is formed by recessing a main housing part 100 to be described below into a shape corresponding to the cleaner body 2100, faces. A direction in which an opposite surface of the main housing part 100 faces with respect to the coupling part 115 can be defined as a rear.

First, a configuration of the cleaner body 2100 of the cleaner 20 according to the present invention will be briefly described.

Referring to FIG. 2, the cleaner body 2100 may include a suction part 2110 for providing a flow path through which air including dust may flow, a dust separation part 2120 communicating with the suction part 2110 to separate dust suctioned into an inside thereof through the suction part 2110, a suction motor 2130 for generating a suction force of suctioning the air, a handle 2140 gripped by a user, and a battery housing 2150 for accommodating a battery therein.

In addition, the cleaner body 2100 may further include a dust bin 2160.

Here, the dust bin 2160 may communicate with the dust separation part 2120 and store dust separated from the dust separation part 2120.

Referring to FIG. 3, the dust bin 2160 may include a discharge cover 2161. The discharge cover 2161 may include a cover body 2161a and a hinge part 2161b. The cover body 2161a may rotate about the hinge part 2161b to open and close the dust bin 2160. The hinge part 2161b may be disposed adjacent to the battery housing 2150. The discharge cover 2161 may be coupled to the dust bin 2160 through hook coupling.

Meanwhile, the dust bin 2160 may further include a coupling lever 2161c. The discharge cover 2161 may be separated from the dust bin 2160 through the coupling lever 2161c. The coupling lever 2161c may be disposed downward based on a state in which the cleaner 20 is coupled to the cleaner station 10. The coupling lever 2161c may elastically deform a hook formed to extend from the cover body 2161a to release the hook coupling between the cover body 2161a and the dust bin 2160 when an external force is applied.

Next, the cleaner station 10 according to the present invention will be described.

Referring to FIG. 2, the cleaner station 10 may include the main housing part 100, a dust collection motor 200, include a dust storage part 300, and may include a sterilization part 400.

The main housing part 100 is a component to which the cleaner 20 is coupled and may form an exterior of the cleaner station 10. Specifically, the main housing part 100 may be formed in a pillar shape including at least one outer wall surface. As an example, the main housing part 100 may be formed in a shape similar to a quadrangular pillar.

The main housing part 100 may have a space formed therein to accommodate the dust collection motor 200, the dust storage part 300, and the sterilization part 400.

The main housing part 100 may include a ground support part 150. In this case, the ground support part 150 may be disposed toward the ground. A bottom surface of the ground support part 150 in contact with the ground may be not only disposed parallel to the ground, but also disposed to be inclined with respect to the ground at a predetermined angle. With this configuration, there is an advantage that the dust collection motor 200 accommodated inside the main housing part 100 can be stably supported and the overall weight can be balanced even when the cleaner 20 is coupled.

In addition, the ground support part 150 may be in the form of a plate formed to extend from a bottom surface of the main housing part 100 to increase an area in contact with the ground in order to prevent the cleaner station 10 from falling over and maintain balance.

The main housing part 100 may include at least one outer wall surface as described above. As an example, the main housing part 100 may include a first outer wall surface 110 on which the coupling part 115 is formed and further include a second outer wall surface 120, a third outer wall surface 130, and a fourth outer wall surface 140 sequentially disposed counterclockwise in a state of facing the first outer wall surface 110.

The coupling part 115 formed on the first outer wall surface 110 may be provided by recessing the first outer wall surface 110 to correspond to a shape of a portion in a direction in which the dust bin 2160 of the cleaner 20 is disposed. With this configuration, the portion of the cleaner 20 may be coupled to the cleaner station 10 and supported by the cleaner station 10.

Some (e.g., the dust storage part 300) of components accommodated in the main housing part 100 may be provided to be opened so as to be exposed. As an example, when viewing the cleaner station 10 from the front, a portion of the left of the first outer wall surface 110 may be opened toward the fourth outer wall surface 140, and a portion of the right of the first outer wall surface 110 may be opened toward the second outer wall surface 120.

Alternatively, as another example, a portion of the first outer wall surface 110 and a portion of the second outer wall surface 120 may be formed integrally and opened together toward the second outer wall surface 120, and a portion of the first outer wall surface 110 and a portion of the fourth outer wall surface 140 may be formed integrally and opened together toward the fourth outer wall surface 140.

The cleaner station 10 according to the present invention may further include a cover opening unit 500.

Referring to FIG. 4, the cover opening unit 500 is provided to open the discharge cover 2161 of the cleaner 20.

The cover opening unit 500 may include a push protrusion 510, a cover opening gear 520, and a cover opening motor (not shown).

The push protrusion 510 may be disposed at a location at which the coupling lever 2161c may be pressed when coupled to the cleaner 20. The push protrusion 510 may linearly reciprocate to press the coupling lever 2161c. Specifically, the push protrusion 510 may be coupled to the cover opening gear 520 and moved together by the movement of the cover opening gear 520.

The cover opening motor may provide power for moving the push protrusion 510 to the cover opening gear 520.

The cover opening gear 520 may be coupled to the cover opening motor and may move the push protrusion 510 using the power of the cover opening motor. More specifically, the cover opening gear 520 may include a first cover opening gear 521 for receiving rotational power from a shaft of the cover opening motor, and a second cover opening gear 522 engaged with the first cover opening gear 521 and transmitting the linear reciprocating movement to the push protrusion 510.

In this case, the first cover opening gear 521 may be configured as a pinion gear, and the second cover opening gear 522 may be configured as a rack gear.

That is, when the body 2100 of the cleaner 20 is fixed to the coupling part 115, the cover opening motor may separate the discharge cover 2161 from the dust bin 2160 by moving the push protrusion 510 through the cover opening gear 520.

The cleaner station 10 according to the present invention may further include a door unit 600.

Referring to FIG. 5, the door unit 600 may include a door 610, a door arm 620, and a door motor 630.

The door 610 may be coupled to the coupling part 115 by a hinge 605 and may rotate about the hinge 605 to open and close an inside and outside of the main housing part 100.

More specifically, in a state in which the door 610 closes the inside of the main housing part 100, when the door arm 620 pulls the door 610, the door 610 may be moved by rotating toward the inside of the main housing part 100 of the cleaner station 10. Meanwhile, when the door arm 620 pushes the door, the door 610 may be moved by rotating toward an outside of the cleaner station 10.

The door motor 630 may provide power for rotating the door 610 to the door arm 620. Specifically, the door motor 630 may rotate the door arm 620 in a forward or reverse direction. Here, the forward direction may be a direction in which the door arm 620 pulls the door 610 toward the inside of the main housing part 100. In addition, the reverse direction may be a direction in which the door arm 620 pushes the door 610 toward the outside of the main housing part 100.

The door arm 620 may connect the door 610 to the door motor 630 and open and close the door 610 using the power generated from the door motor 630.

As an example, the door arm 620 may include a first door arm 621 and a second door arm 622. One end portion of the first door arm 621 may be coupled to the door motor 630. The first door arm 621 may be rotated by the power of the door motor 630. The other end portion of the first door arm 621 may be rotatably coupled to the second door arm 622. The first door arm 621 may transmit a force transmitted from the door motor 630 to the second door arm 622. One end portion of the second door arm 622 may be coupled to the first door arm 621. The other end portion of the second door arm 622 may be coupled to the door 610. The second door arm 622 may push or pull the door 610.

The cleaner station 10 according to the present invention may further include a suction flow path 700.

Here, the suction flow path 700 is a passage through which air including dust may flow. Therefore, when the discharge cover 2161 is separated from the dust bin 2160, the dust inside the dust bin 2160 may be collected into the dust storage part 300 to be described below through the suction flow path 700.

Meanwhile, the dust collection motor 200 may be accommodated inside the main housing part 100 and disposed under the dust storage part 300. The dust collection motor 200 may generate a suction force in the suction flow path 700. Therefore, the dust collection motor 700 may suction dust inside the dust bin 2160 of the cleaner 20.

Next, the dust storage part 300 is a component that is accommodated inside the main housing part 100 and collects dust suctioned from the inside of the dust bin 2160 of the cleaner 20 by the dust collection motor 200.

The dust storage part 300 may be detachably coupled to the main housing part 100.

Therefore, when the main housing part 100 is opened, the dust storage part 300 may be discarded by being separated from the main housing part 100, and a new dust storage part 300 may be coupled to the main housing part 100. That is, the dust storage part 300 can be defined as a consumable part.

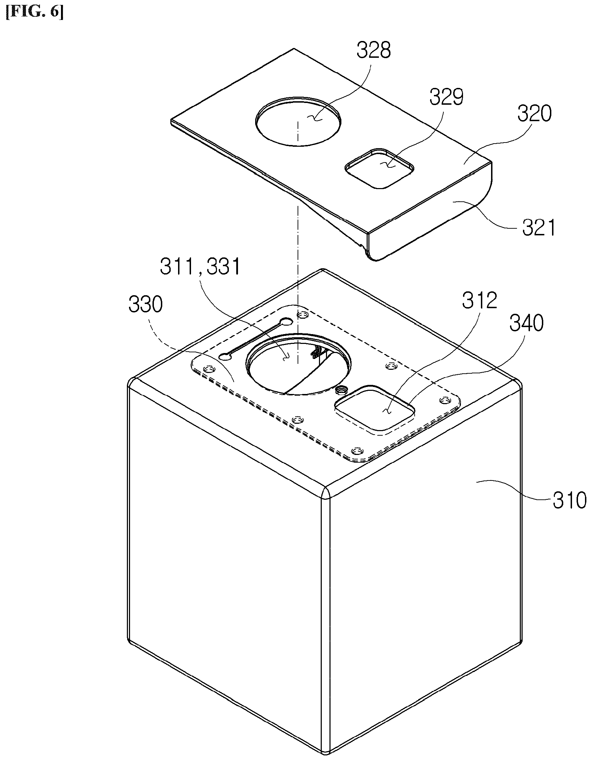

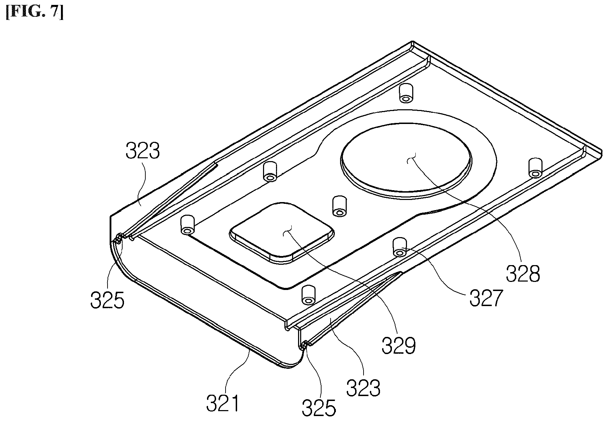

FIG. 6 is an exploded view of main components of a dust storage part 300 according to the present invention, and FIG. 7 is an enlarged view of an upper plate 320 of the dust storage part 300 according to the present invention.

The dust storage part 300 may include the dust bag 310 and the upper plate 320.

The dust bag 310 is a component for accommodating the dust suctioned from the cleaner 20 and storing the same therein.

The dust bag 310 may be provided so that dust is accommodated therein as a volume is increased when a suction force is generated by the dust collection motor 200. To this end, the dust bag 310 may be made of a material that allows air to pass therethrough but does not allow a foreign substance such as dust to pass therethrough. As an example, the dust bag 310 may be made of a non-woven material and may have a hexahedral shape based on when the volume is increased.

When an airflow is formed by the suction force of the dust collection motor 200, air including the foreign substance, which flows inside the dust bin 2160 of the cleaner, moves to the dust bag 310 through the suction flow path 700, only the foreign substance is left in the dust bag 310, and the air exits the dust bag 310.

The upper plate 320 is a component configured to be coupled to an outer upper surface of the dust bag 310 and inserted into fixed holders 810 and 830 of a dust storage housing part 800 to be described below. The upper plate 320 may be inserted into installation grooves 811 and 831 of the fixed holders 810 and 830 in a sliding manner. With respect to the upper plate 320, the insertion is made from the rear to the front of the upper plate 320.

Sliding surfaces inserted into the installation grooves 811 and 831 of the upper plate 320 may be provided in a rectangular shape. In addition, when the upper plate 320 is inserted into the dust storage housing part 800, the upper plate 320 may be inserted by sliding from the first outer wall surface 110 side to the third outer wall surface 130 side of the main housing part 100.

The upper plate 320 may include a dust bag handle 321.

The dust bag handle 321 is provided at a front of the dust storage part 300. The dust bag handle 321 may be gripped by a user when the dust storage part 300 is separated from or inserted into the dust storage housing part 800. The sliding surface of the upper plate 320 and the dust bag handle 321 may be connected at a right angle. The user may grip the dust bag handle 321 and push the sliding surface into the installation grooves 811 and 831. The user may grip the dust bag handle 321 and take the sliding surface out of the installation grooves 811 and 831.

The upper plate 320 may include an inclined rib 323.

The inclined rib 323 is a component that is formed to protrude and extend backward from both left and right end portions of the dust bag handle 321. At the same time, the inclined rib 323 is a component that is formed to protrude and extend downward from a lower surface of the sliding surface. The inclined rib 323 may be independently provided at each of the left and right end portions of the dust bag handle 321. The inclined rib 323 may be formed to extend to have a rearward and upward inclination.

The dust storage part 300 may further include a lower plate 330.

The lower plate 330 is a component that is coupled to an inner upper surface of the dust bag 310. The lower plate 330 may be provided in a rectangular shape. The lower plate 330 may be coupled to the upper plate 320 through a separate fastening means 327 with the dust bag 310 interposed therebetween.

A transmissive panel 340 may be coupled to the lower plate 330.

When the dust storage part 300 is coupled to the inside of the main housing part 100, the transmissive panel 340 is disposed at a location corresponding to a location at which the sterilization part 400 to be described below is disposed. That is, when the upper plate 320 is inserted into the dust storage housing part 800, the sterilization part 400 and the transmissive panel 340 are disposed to face each other.

In addition, the transmissive panel 340 is made of a material that allows sterilization light emitted from the sterilization part 400 to transmit toward an inside of the dust bag 310. As an example, the transmissive panel 340 may be made of a poly methyl methacrylate (PMMA) material.

In a suction process of suctioning dust into the dust bag 310 by a strong suction force, the flying dust swirls inside the dust bag 310 in all directions. Even when the dust suction process is finished and the dust swirl subsides inside the dust bag 310, a fine dust floating phenomenon may occur inside the dust bag 310. Therefore, dust continuously stick to a lower surface of the transmissive panel 340 facing the inside of the dust bag 310. This reduces a transmittance of the transmissive panel 340 over time, thereby lowering the sterilization efficiency of the sterilization part 400.

Since the cleaner station 10 according to the present invention has the dust storage part 300 detachably connected to the inside of the cleaner station 10 and the transmissive panel 340 for transmitting sterilization light is included in the dust storage part 300, the transmissive panel 340 as well as the dust bag 310 may be replaced as a consumable part. That is, since the transmissive panel 340 to which dust sticks may be periodically replaced with a new product, a reduction in the transmittance of the transmissive panel 340 can be prevented, and the sterilizing performance of the sterilization part 400 can be maintained without degradation.

Meanwhile, a dust hole 328 through which air including dust is introduced is formed in the upper plate 320. The dust hole 328 communicates with a flow path connection member 870 to be described below. The flow path connection member 870 is connected to the suction flow path 700, and as a result, air flowing through the suction flow path 700 passes through the flow path connection member 870 and the dust hole 328 and flows into the inside of the dust bag 310.

A sterilization hole 329 is formed in the upper plate 320 so that sterilization light emitted from the sterilization unit 400 may pass therethrough. A light source of the sterilization unit 400 is disposed above the sterilization hole 329 and the dust bag 310 is disposed thereunder so that the inside of the dust bag 310 is sterilized by the sterilization light emitted by the light source.

In an embodiment shown in FIGS. 6 and 7, the dust hole 328 and the sterilization hole 329 are shown as being circular and rectangular, respectively, but the shapes are not limited to the shown embodiments as long as the above-described functions are maintained.

The lower plate 330 is also formed with a dust hole 331 through which air including dust is introduced like the upper plate 320. The dust hole 331 of the lower plate 330 communicates with the dust hole 328 of the upper plate 320.

The upper surface of the dust bag 310 is also formed with the dust hole 311 through which air including dust is introduced like the upper plate 320 and the lower plate 330. The dust hole of the dust bag 310 communicates with the dust hole 328 of the upper plate 320 upward and communicates with the dust hole 331 of the lower plate 330 downward.

The upper surface of the dust bag 310 is formed with a sterilization hole 312 so that the sterilization light emitted from the sterilization part 400 may pass therethrough. The sterilization hole 329 of the upper plate 320 is disposed above the sterilization hole 312 of the dust bag 310, and the transmissive panel 340 is disposed thereunder.

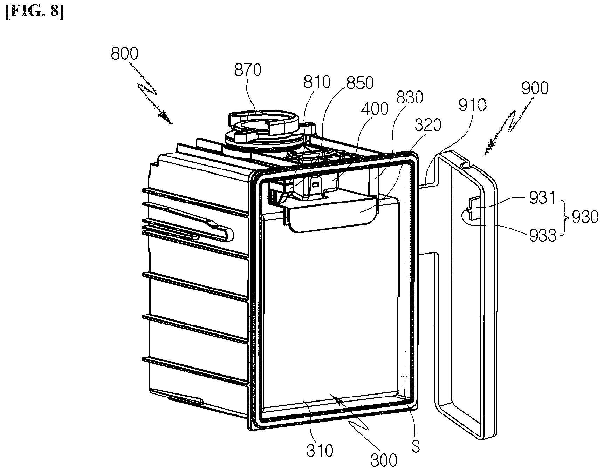

FIG. 8 is a view showing a state in which the dust storage part 300 has been accommodated in the dust storage housing part 800 according to the present invention.

The cleaner station 10 according to the present invention further includes the dust storage housing part 800.

An accommodation space S in which the dust storage part 300 is accommodated is formed inside the dust storage housing part 800. The dust storage housing part 800 is provided so that the dust storage part 300 may be detachably attached.

In the embodiment described herein, an example in which the dust storage housing part 800 is accommodated in a partial area of an internal space of the main housing part 100 and the dust storage part 300 is accommodated in the internal accommodation space S of the dust storage housing part 800 will be described. That is, the dust storage housing part 800 may be provided separately from the main housing part 100. However, the embodiment is not limited thereto, and the main housing part 100 and the dust storage housing part 800 may be separate components, and the dust storage housing part 800 may form a portion of the main housing part 100.

The dust storage housing part 800 may have a hexahedral shape with an open one surface as shown in FIG. 8. The dust storage part 300 may be coupled to or separated from the dust storage housing part 800 through the open one surface.

A direction in which the open one surface of the dust storage housing part 800 faces is defined as a front, and one surface to which the suction flow path 700 is connected is defined as a top.

The dust storage housing part 800 may communicate with the suction flow path 700 through a flow path connection member 870 provided on one top surface (upper surface). The flow path connection member 870 may have one end connected to the suction flow path 700 and the other end connected to the dust storage part 300.

Therefore, the dust suctioned by the cleaner 20 may move to the dust storage part 300 through the suction flow path 700 and the flow path connection member 870.

The dust storage housing part 800 includes the fixed holders 810 and 830.

The fixed holders 810 and 830 are components for guiding the dust storage part 300 to be inserted into the accommodation space S of the dust storage housing part 800. The fixed holders 810 and 830 are components for supporting the dust storage part 300 so as not to shake in a state in which the dust storage part 300 is inserted into the accommodation space S. In this case, the insertion may be made by sliding the dust storage part 300 into the installation grooves 811 and 831 provided in the fixed holders 810 and 830.

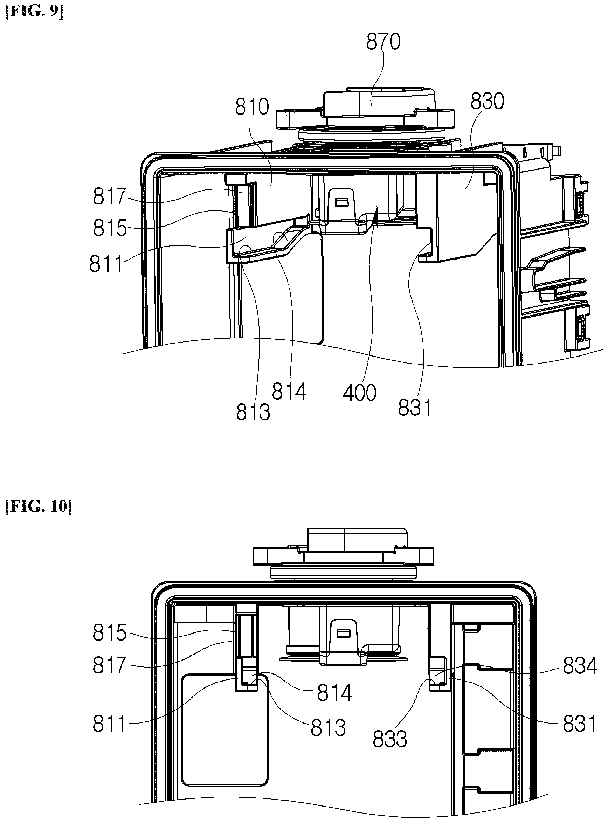

The fixed holders 810 and 830 may be fixedly disposed on an upper surface of the accommodation space S of the dust storage housing part 800. A more detailed structure of the fixed holders 810 and 830 will be described below with reference to FIGS. 9 and 10.

The dust storage housing part 800 includes a stopper 850.

The stopper 850 is connected to the fixed holders to linearly reciprocate a predetermined distance. A direction in which the stopper 850 linearly reciprocates is determined according to the insertion or separation of the dust storage part 300 into or from the dust storage housing part 800.

When the dust storage part 300 is inserted into the dust storage housing part 800, the stopper 850 may move to a first location. When the dust storage part 300 is separated from the dust storage housing part 800, the stopper 850 may move to a second location opposite to the first location.

More specifically, when the stopper 850 slides in a direction in which the dust storage part 300 is inserted into the installation grooves 811 and 831, the stopper 850 may be pushed while being supported by a partial area of the dust storage part 300 and may linearly move to the first location.

When the stopper 850 slides in a direction in which the dust storage part 300 is separated from the installation grooves 811 and 831, the stopper 850 may linearly move to the second location interfering with a partial area of the housing cover part 900 to be described below as the support by the dust storage part 300 is released.

In a possible embodiment, the linear movement between the first location and the second location may be a vertical movement. More specifically, the linear movement from the first location to the second location may be a downward movement due to gravity, and the linear movement from the second location to the first location may be an upward movement pushed by the dust storage part 300 upward.

Alternatively, in a possible embodiment, the linear movement between the first location and the second location may be a horizontal movement.

In any of the above-described embodiments, the movement of the stopper 850 from the first location to the second location is a linear movement in a direction in which the housing cover part 900 interferes with the stopper 850. In the present specification, an embodiment in which the linear reciprocating movement of the stopper 850 is the vertical movement will be described.

The cleaner station according to the present invention may further include the sterilization part 400.

The sterilization part 400 may be coupled to the upper surface of the dust storage housing part 800.

The sterilization part 400 is a component that is provided to sterilize dust collected in the dust storage part 300. The sterilization part 400 may include a light source for emitting sterilization light and a protective panel disposed under the light source to protect the light source.

In a possible embodiment, the light source and the protective panel may be coupled to the dust storage housing part 800 in a form that is accommodated in a separately provided sterilization part 400 housing. Alternatively, in a possible embodiment, the light source and the protective panel may be coupled to the dust storage housing part 800 in a form that is accommodated in a space formed by bending a partial area of the upper surface of the dust storage housing part 800.

The coupling form of the sterilization part 400 and the dust storage housing part 800 is not limited to any one embodiment as long as the light source of the sterilization part 400 is disposed to emit sterilization light toward the dust storage part 300.

Here, the light source may include at least one light emitting diode (LED) capable of emitting sterilization light having sterilization power capable of removing bacteria. The sterilization light emitted by the light source may have a wavelength that varies depending on the type of the LED.

For example, the light source may be an LED that emits ultraviolet light having a UV-C wavelength range. The ultraviolet light is classified into UV-A (315 nm to 400 nm), UV-B (280 nm to 315 nm), and UV-C (200 nm to 280 nm) depending on a wavelength, and among them, ultraviolet light in the UV-C range can damage a DNA double helix of microorganisms and suppress the proliferation of the microorganisms.

Alternatively, as another example, the light source may be an LED that emits visible light having a wavelength of 405 nm. Blue light having a wavelength of 405 nm has a wavelength in a boundary area between visible light and ultraviolet light and has been proven to have sterilization power.

The protective panel may be disposed to be spaced a predetermined distance from the light source to prevent damage to the light source. In this case, the protective panel may be made of a material that maximizes the transmittance of the light source. As an example, the protective panel may be made of quartz. It is known that quartz does not interfere with the transmission of ultraviolet light in the UV-C area.

The cleaner station 10 according to the present invention has an advantage that by providing the sterilizing part 400, the cleaner station 10 can be hygienically managed even when the dust suctioned from the dust bin 2160 of the cleaner 20 is stored in the dust storage part 300 for a long time.

In FIG. 8, the sterilization part 400 is shown as being disposed in front of the flow path connection member 870, but in a possible embodiment, the sterilization part 400 may be disposed behind the flow path connection member 870. Alternatively, in a possible embodiment, the sterilization part 400 may be disposed at a left or right side of the flow path connection member 870.

Alternatively, in a possible embodiment, the sterilization part 400 may be disposed to emit sterilization light toward the center of the upper surface of the dust storage part 300. In this embodiment, it is possible to minimize a dead zone that is a range in which the sterilization light does not reach the dust bag 310.

The cleaner station 10 according to the present invention further includes the housing cover part 900.

The housing cover part 900 is provided to open and close a partial area of the dust storage housing part 800. More specifically, the housing cover part 900 may be connected to the dust storage housing part 800 at the open side surface of the dust storage housing part 800 by a hinge member 910. The housing cover part 900 may open or close the open one surface while rotating about a hinge axis of the hinge member 910.

In the embodiment of FIG. 8, the hinge member 910 is shown as being connected to the right side of the dust storage housing part 800, but in a possible embodiment, the hinge member 910 may be connected to the left side of the dust storage housing part 800. Alternatively, in a possible embodiment, the hinge member 910 may be connected to an upper side of the dust storage housing part 800. Alternatively, in a possible embodiment, the hinge member 910 may be connected to a lower side of the dust storage housing part 800.

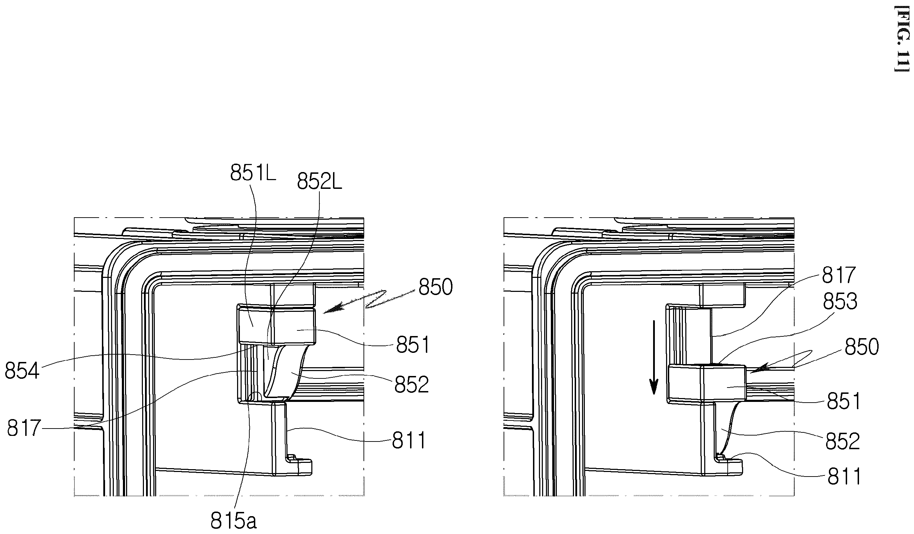

The housing cover part 900 may include an interference member 930 that protrudes in a direction toward the inside of the dust storage housing part 800. The interference member 930 is a component that interferes with the stopper 850 to be described below to prevent the housing cover part 900 from closing the dust storage housing part 800. The stopper 850 will be described below with reference to FIGS. 11 to 13. The interference member 930 is disposed at a location at which it may interfere with the stopper 850 or may be released from an interference state depending on the location of the stopper 850.

More specifically, the interference member 930 interferes with the stopper 850 at a location of the stopper 850 in a state in which the dust storage part 300 has been separated from the dust storage housing part 800. The interference member 930 does not interfere with the stopper 850 at a location of the stopper 850 in a state in which the dust storage part 300 has been inserted into the dust storage housing part 800.

The interference member 930 may include a first interference member 931 and a second interference member 933. The first interference member 931 may protrude a predetermined length toward the inside of the dust storage housing part 800. The second interference member 933 may be connected to the first interference member 931 and may further protrude from the first interference member 931 toward the inside of the dust storage housing part 800.

In the embodiment (embodiment of FIG. 8) in which the interference member 930 includes the first interference member 931 and the second interference member 933, when the dust storage part 300 is separated from the dust storage housing part 800, the second interference member 933 interferes with the stopper 850. A connection portion where a lower surface of the first interference member 931 and the second interference member 933 are vertically connected may be disposed to correspond to a connection portion where the sliding surface of the upper plate 320 and the dust bag handle 321 are vertically connected. As a result, the first interference member 931 may serve to push the dust bag handle 321 to the correct location, which will be described below, when the dust storage part 300 is not fully inserted into the dust storage housing part 800.

FIG. 9 is an enlarged view of the fixed holders 810 and 830 according to the present invention, and FIG. 10 is a front view of the fixed holders 810 and 830 according to the present invention.

The fixed holders 810 and 830 includes the first fixed holder 810 disposed at an upper left side of the accommodation space S and the second fixed holder 830 disposed at an upper right side thereof. The sterilization unit 400 may be disposed between the first fixed holder 810 and the second fixed holder 830. The first and second fixed holders 810, 830 are provided so that their respective upper surfaces are fixed to the upper surface of the accommodation space S.

That is, the fixed holders 810 and 830 are fixed components that do not move in any direction and may support the dust storage part 300 so as not to shake in the accommodation space S.

The first and second fixed holders 810 and 830 may include the installation grooves 811 and 831 formed so that the dust storage part 300 slides. The installation grooves 811 and 831 may extend in a front-rear direction so that the dust storage part 300 may be inserted from the front to the rear. The installation groove 811 of the first fixed holder 810 may mean a concave structure in which a right surface of the first fixed holder 810 is recessed leftward at a predetermined depth and height. The installation groove 831 of the second fixed holder 830 may mean a concave structure in which a left surface of the second fixed holder 830 is recessed rightward at a predetermined depth and height.

The first and second fixed holders 810 and 830 may further include first installation surfaces 813 and 833 that are disposed on a path along which the dust storage part 300 slides and horizontally extend, and second installation surfaces 814 and 834 that are connected to rear ends of the first installation surfaces 813 and 833 and extend to have a rearward and upward inclination. The first installation surfaces 813 and 833 and the second installation surfaces 814 and 834 can define lower surfaces of the installation grooves 811 and 831.

Therefore, the dust storage part 300 is inserted horizontally along only the first installation surfaces 813 and 833 in an initial section in which the dust storage part 300 slides along the installation grooves 811 and 831 and is then inserted while being pushed upward along the inclinations of the second installation surfaces 814 and 834.

In a state in which the dust storage part 300 has been separated from the installation grooves 811 and 831 or a state in which the dust storage part 300 has been inserted into only the first installation surfaces 813 and 833 of the installation grooves 811 and 831, the stopper 850 and the interference member 930 are in contact with each other to interfere with the closing of the dust storage housing part 800. In a state in which the dust storage part 300 has been inserted into the second installation surfaces 814 and 834 of the installation grooves 811 and 831, the stopper 850 and the interference member 930 do not interfere with the closing of the dust storage housing part 800.

The fixed holder further includes a rail groove 815 that provides a space in which the stopper 850 moves. The rail groove 815 may mean a concave structure that is formed to be recessed from the front to the rear of the fixed holder at a predetermined depth and height.

As in the embodiments shown in FIGS. 9 and 10, the rail groove 815 may be provided only in the first fixed holder 810. Alternatively, in another possible embodiment, the rail groove 815 may be provided only in the second fixed holder 830. As described above, when the rail groove 815 is provided only in any one fixed holder, the first fixed holder 810 and the second fixed holder 830 are provided to have different shapes.

Alternatively, in another possible embodiment, the rail groove 815 may be provided in both the first fixed holder 810 and the second fixed holder 830. As described above, when the rail groove 815 is provided in both left and right fixed holders 810 and 830, two stoppers 850 may also be provided and coupled to the first fixed holder 810 and the second fixed holder 830, respectively. When the rail groove 815 is provided in both left and right fixed holders 810 and 830, the first fixed holder 810 and the second fixed holder 830 are provided to have symmetrical shapes.

In the present specification, a structure in which the rail groove 815 is formed in the vertical direction as shown in FIG. 9 will be described. A lower end portion of the rail groove 815 may be connected to an upper end portion of the installation groove 811.

The fixed holder 810 may further include a guide rail 817 for guiding the stopper 850 to move linearly. The guide rail 817 may be formed to extend in the vertical direction on a rear surface of the rail groove 815. That is, a lower end portion of the guide rail 817 may extend to the lower end portion of the rail groove 815 and may be connected to the upper end portion of the installation groove 811. An upper end portion of the guide rail 817 may extend to an upper end portion of the rail groove 815.

The fixed holder 810 may further include a support surface 815a that stops the downward movement by supporting a partial surface of the stopper 850 at the lower end portion of the rail groove 815.

The support surface 815a can define a lower surface of the rail groove 815. The support surface 815a may be connected to the installation grooves 811 and 831. The support surface 815a may be connected to the lower end portion of the guide rail 817. The downward movement of the stopper 850 is stopped by the support surface 815a.

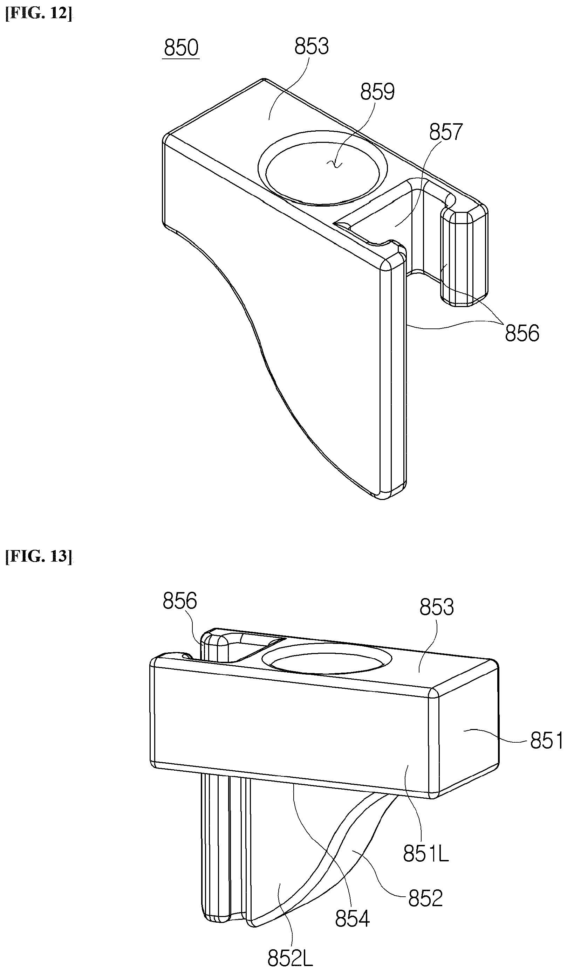

FIG. 11 is a view showing a state in which the stopper 850 coupled to the fixed holders 810 and 830 is located at each of a first location and a second location according to the present invention, FIG. 12 is a perspective view of the stopper 850 when viewed from the rear according to the present invention, and FIG. 13 is a perspective view of the stopper 850 when viewed from the front according to the present invention.

The stopper 850 may include a rail insertion groove 857 into which the guide rail 817 is inserted.

A direction in which one surface of the rail insertion groove 857 is provided can be defined as a rear of the stopper 850, and an opposite direction can be defined as a front thereof. The rail insertion groove 857 may mean a concave structure in which the entire rear surface of the stopper 850 is recessed forward at a predetermined depth and width. The depth and width of the rail insertion groove 857 may be formed to a size in which the guide rail 817 may be inserted.

The stopper 850 may include a hook 856 that is coupled by being caught on the guide rail 817.

The hook 856 is a component that protrudes and extends from a rear end of the rail insertion groove 857 to the center of the stopper 850. The hook 856 is formed at each of rear ends of left and right sides of the rail insertion groove 857. The hook 856 is coupled by being caught on a protruding step of the guide rail 817 in a state in which the guide rail 817 has been inserted into the rail insertion groove 857.

Therefore, the stopper 850 may linearly reciprocate in the vertical direction in a state of being coupled to the guide rail 817.

In this case, a location of the stopper 850 in a state in which the upper end portion of the rail groove 815 is in contact with the upper surface 853 of the stopper 850 as the stopper 850 moves upward along the guide rail 817 can be defined as the first location (see the left of FIG. 11).

In addition, a location of the stopper 850 in a state in which the support surface 815a of the fixed holders 810 and 830 is in contact with a support surface 854 of the stopper 850 as the stopper 850 moves downward along the guide rail 817 can be defined as the second location (see the right of FIG. 11).

The stopper 850 may include a guide inclined surface 852 having an inclination in the insertion direction of the upper plate 320.

The guide inclined surface 852 can define a front partial area of the stopper 850. More specifically, when a surface perpendicular to the upper surface 853 of the stopper 850 is defined as the front surface 851 of the stopper 850, a surface that is connected to the front surface 851 and has an inclination in the insertion direction of the upper plate 320 becomes the guide inclined surface 852. That is, when the upper plate 320 is inserted from the front to the rear, the guide inclined surface 852 has a rearward and downward inclination.

In the embodiment in which the first fixed holder 810 is coupled to the stopper 850, a left surface 851L connected to the front surface 851 of the stopper 850 may form a step with a left surface 852L connected to the guide inclined surface 852. A connection surface between the left surfaces 851L and 852L forming the step can be defined as the stopper support surface 854. The stopper support surface 854 is a surface in contact with the support surface 815a of the fixed holders 810 and 830 when the stopper 850 moves downward as described above.

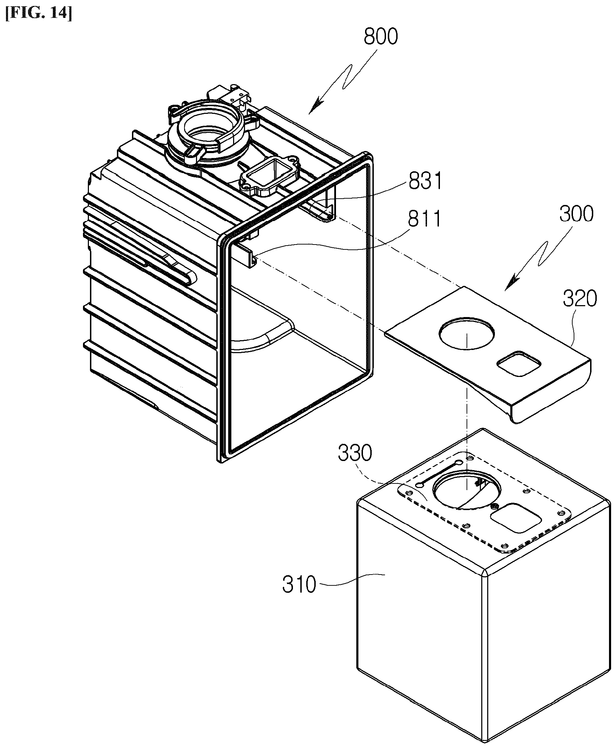

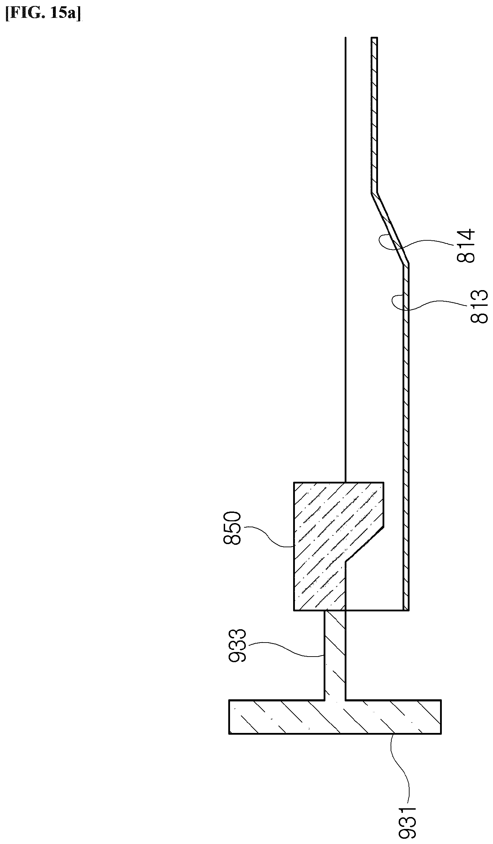

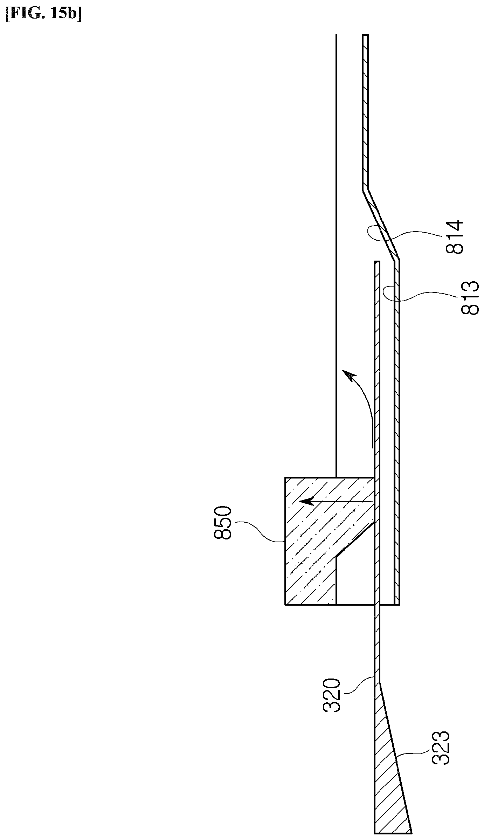

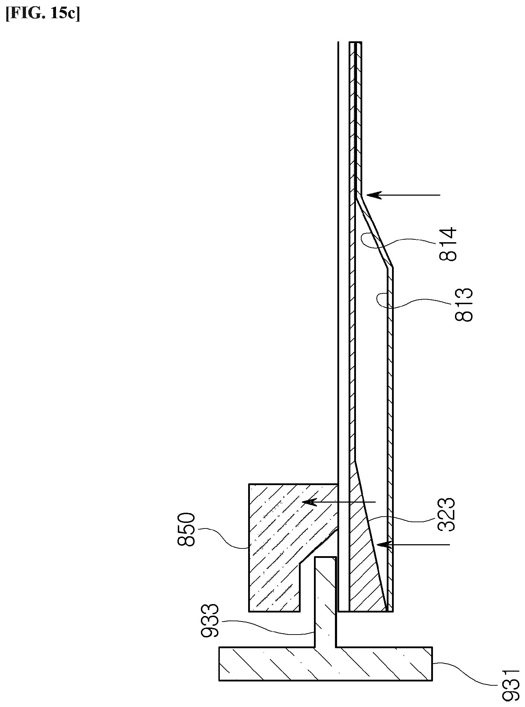

Hereinafter, an operation of the dust bag recognition structure according to the present invention will be described in detail further with reference to FIGS. 14 and 15.

FIG. 14 is a perspective view separately showing main components to describe a coupling relationship between the dust storage part 300 and the fixed holders 810 and 830 according to the present invention, and FIG. 15 is a conceptual diagram for sequentially describing a process in which the dust storage part 300 is inserted into the dust storage housing part 800 and a movement path of the stopper 850 accordingly according to the present invention.

When the upper plate 320 of the dust storage part 300 is not inserted into the installation grooves 811 and 831 of the fixed holders 810 and 830 (i.e., when the dust storage part 300 is fully separated from the dust storage housing part 800, the stopper 850 moves downward to the second location (see FIG. 15A).

In this case, when a user closes the housing cover part 900 to close the dust storage housing part 800, the stopper 850 and the housing cover part 900 interfere with each other in a state in which the housing cover part 900 is not yet fully closed.

More specifically, when the stopper 850 moves downward to the second location, as the second interference member 933 of the housing cover part 900 is in contact with a front surface of the stopper 850, the second interference member 933 may not enter the accommodation space S of the dust storage housing part 800 any more. That is, the housing cover part 900 and the stopper 850 interfere with each other so that the dust storage housing part 800 is not closed.

Therefore, the user may recognize that the second interference member 933 is in contact with the stopper 850 and recognize the absence of the dust storage part 300.

When the upper plate 320 of the dust storage part 300 is inserted into the installation grooves 811 and 831 of the fixed holders 810 and 830, the upper plate 320 at the beginning of the insertion slides toward an inside of the accommodation space S along the guide inclined surface 852 (see FIG. 15B).

In this case, the stopper 850 is pushed while being supported by the upper plate 320, and the stopper 850 is in a state of being coupled to the guide rail 817 to enable only the linear movement in the vertical direction, and thus performs the upward movement that moves upward by being pushed.

Meanwhile, since the guide inclined surface 852 has a downward inclination along the rear that is the insertion direction of the upper plate 320, it can be easy to achieve the insertion of the upper plate 320 and the upward movement of the stopper 850 with a small force.

When the upper plate 320 of the dust storage part 300 is further inserted into the installation grooves 811 and 831 of the fixed holders 810 and 830, that is, when the upper plate 320 is inserted into the second installation surfaces 814 and 834 through the first installation surfaces 813 and 833, the rear end of the upper plate 320 moves upward along the inclinations of the second installation surfaces 814 and 834. When the upper plate 320 is continuously inserted and the inclined rib 323 reaches the first installation surfaces 813 and 833, the upper plate 320 moves upward along the inclination of the inclined rib 323.

When the upper plate 320 is fully inserted, the stopper 850 is in a state of moving upward to the first location (see FIG. 15C).

Meanwhile, since the second installation surfaces 814 and 834 have a rearward and upward inclination, it can be easy to achieve the upward movement of the upper plate 320 and the stopper 850 with a small force. Since the upper plate 320 also has the inclined rib 323 having a rearward and upward inclination, it can be easy to achieve the upward movement of the upper plate 320 and the stopper 850 with a small force through cooperation therebetween.

Referring to FIG. 15C, since the guide inclined surface 852 has a rearward and downward inclination, when the housing cover part 900 is closed in a state in which the stopper 850 has moved upward to the first location, the second interference member 933 may enter as much as an empty space formed in front of the stopper 850.

Therefore, the housing cover part 900 may fully close the dust storage housing part 800 without any obstruction.

Meanwhile, the stopper 850 is in a state of being supported by the upper plate 320 at the first location. In this case, when the dust storage part 300 is separated from the dust storage housing part 800, that is, when the upper plate 320 supporting the stopper 850 is removed, the stopper 850 moves downward back to the second location. In this case, as described above, the downward movement may be a movement by gravity. Since the stopper 850 is moved downward by gravity and the stopper 850 and the guide rail 817 are coupled, the stopper 850 is prevented from being separated in a different direction.

In addition, since the downward movement of the stopper 850 is stopped at the second location by the support surface 815a of the fixed holders 810 and 830, the stopper 850 is prevented from being separated downward more than the second location.

Therefore, the dust bag 310 recognition structure according to the present invention is implemented in a simple structure that does not require a separate component other than the stopper 850. Such a structure has an effect of reducing the cost of parts and at the same time, has an advantage of being free from various causes of failure compared to an embodiment in which a large number of parts are used.

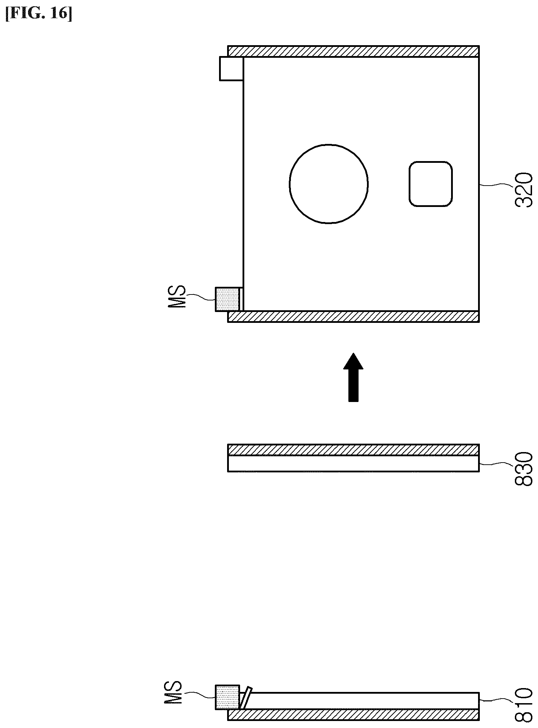

FIG. 16 is a view for describing an embodiment in which a micro switch MS is provided according to the present invention.

The cleaner station 10 according to the present invention may include the micro switch MS.

The micro switch MS may be in contact with the dust storage part 300 to detect whether the dust storage part 300 has been disposed at a correct location when inserted into the dust storage housing part 800. To this end, the micro switch MS may be disposed at a rear end of the installation groove 811 of the first fixed holder 810 or the installation groove 831 of the second fixed holder 830.

The micro switch MS is a well-known electrical switch, which is also known as a limit switch. The micro switch MS is turned on when an operation lever is pressed by a mechanical operation of another component.

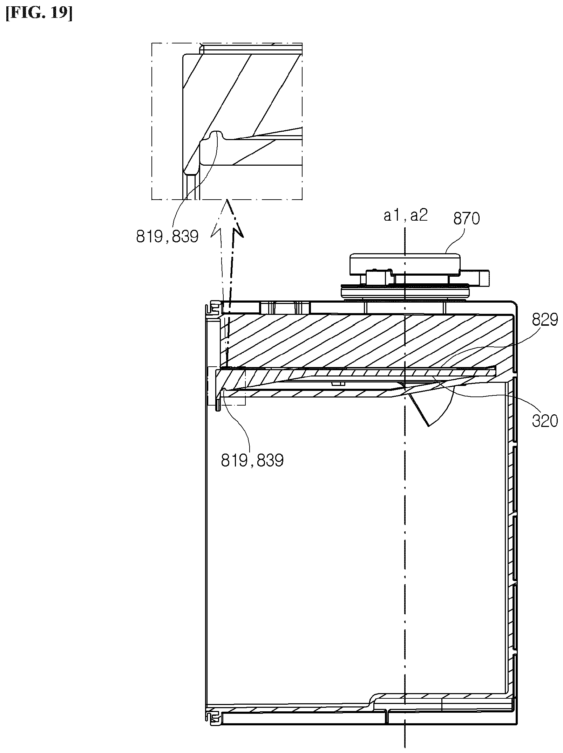

Meanwhile, when the dust storage part 300 is disposed at the correct location, it means that an axis a1 of the flow path connection member 870 and a center axis a2 of the dust hole 311 of the dust bag 310 (or the dust hole 328 of the upper plate 320) are coaxial (see FIG. 19). When the dust storage part 300 is not disposed at the correct location, the suctioned dust may leak to a connection gap between the flow path connection member 870 and the dust hole 328 of the upper plate 320. That is, complete sealing is not achieved, and dust may fly, thereby contaminating the inside of the cleaner station 10.

Referring to FIG. 16, when the dust storage part 300 is disposed at the correct location, the upper plate 320 presses the operation lever of the micro switch MS. In this case, the micro switch MS is turned on. When the dust storage part 300 is not inserted or is not disposed at the correct location, the upper plate 320 does not press the operation lever of the micro switch MS, and thus the micro switch MS remains in an off state.

In a possible embodiment, the cleaner station 10 may be controlled so that the dust collection motor 200 operates only when the micro switch MS is turned on. In a possible embodiment, the cleaner station 10 may generate a warning alarm to check the location of the dust storage part 300 when a user's manipulation command is input in a state in which the micro switch MS has been turned off. The warning alarm may be displayed as a visual signal and/or transmitted as an audible signal.

Therefore, there is an advantage that the user can recognize a state in which the dust storage part 300 has not been completely sealed with the suction flow path 700. In addition, this prevents the inside of the cleaner station 10 from being contaminated with flying dust.

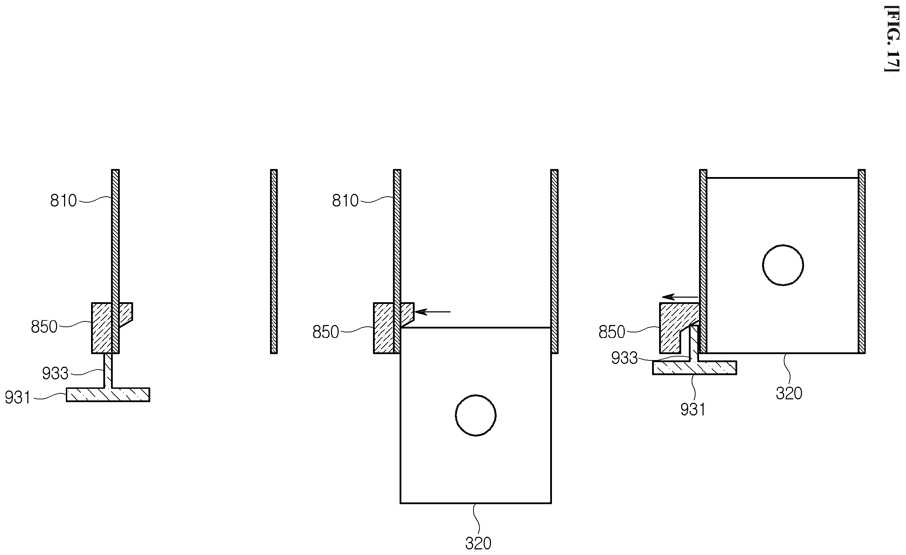

FIG. 17 is a view for describing another embodiment in which a dust bag recognition structure is possible according to the present invention.

Although all components have been described above based on the case where the linear movement of the stopper 850 is the vertical movement, as another embodiment, the linear movement between the first location and the second location of the stopper 850 may be a horizontal movement.

A principle of operation is the same as the case of the vertical linear movement, but the stopper 850 may be disposed in a state of rotating 90 degrees clockwise (when coupled to the second fixed holder 830) or counterclockwise (when coupled to the first fixed holder 810) based on the embodiment of FIG. 11.

Of course, in this embodiment, the guide rail 817 has a shape that extends in the left-right direction rather than the vertical direction. The guide inclined surface 852 of the stopper 850 still has an inclination in the sliding direction of the dust storage part 300.

In a state in which the dust storage part 300 has not been inserted, the interference member 930 of the housing cover part 900 interferes with the front surface of the stopper 850 at the second location.

When the dust storage part 300 is inserted, the stopper 850 is pushed toward the first location while being supported by the side surface of the upper plate 320.

When the dust storage part 300 is fully inserted and the stopper 850 is located at the first location, the interference member 930 of the cover housing may enter the empty space formed in front of the guide inclined surface 852 to close the housing cover part 900.

However, in the embodiment in which the stopper 850 linearly moves in the horizontal direction, there is a need for a separate component for applying an external force toward the second location so that the stopper 850 returns from the first location to the second location.

In a possible embodiment, a spring (not shown) for applying an elastic restoring force toward the second location may be coupled to the stopper 850. That is, in this embodiment, the linear movement from the first location to the second location may be a movement by the elastic restoring force of the spring.

Meanwhile, in the embodiment in which the stopper 850 moves in the vertical direction, the linear movement between the first location and the second location may be achieved by only the action of gravity, but even in this case, the spring may be coupled to the stopper 850. That is, the downward movement from the first location to the second location may also be achieved by the movement by the elastic restoring force of the spring.

In the embodiment of the stopper 850 to which the spring is coupled, a spring coupling hole 859 to which the spring is coupled may be provided in one surface of the stopper 850 (see FIG. 12).

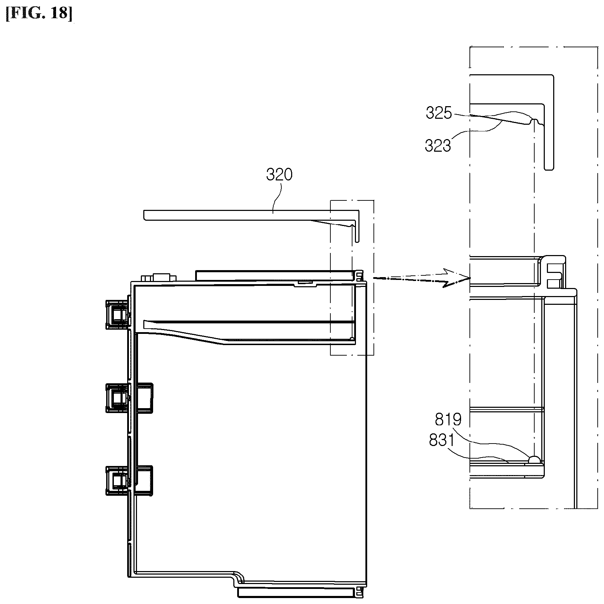

FIG. 18 is a view for describing a correction location installation detection structure of the dust bag 310 according to the present invention, and FIG. 19 is a cross-sectional view showing a state in which the dust bag 310 has been installed at the correct location in the embodiment of FIG. 18.

One sides of the installation grooves 811 and 831 of the fixed holders 810 and 830 and the dust storage part 300 may each be provided with a convex portion and the other sides may each be provided with a concave portion having a shape corresponding to the convex portion. In this case, a concave portion or convex portion provided on the upper plate 320 may be provided on one end portion of the sliding movement path. In a possible embodiment, the one end portion may be a front end portion of the sliding movement path. In another possible embodiment, the one end portion may be a rear end portion of the sliding movement path.

Preferably, it is preferable that the convex portion and the concave portion are provided on the front end portion of the sliding movement path to be close to the dust bag handle 321. This is because whether the convex portion and the concave portion are coupled can be more easily recognized by the user's touch at this location.

More specifically, describing based on the embodiment of FIG. 18, convex portions 819 and 839 are provided in the installation grooves 811 and 831 of the fixed holders 810 and 830, and the concave portion 325 is provided in the upper plate 320 of the dust storage part 300. The convex portions 819 and 839 may be provided on front end portions of the installation grooves 811 and 831. In this case, the concave portion 325 may be provided on a front lower end portion of the inclined rib 323 of the upper plate 320.

In another possible embodiment, the convex portions 819 and 839 may be provided on the front lower end portion of the inclined rib 323 of the upper plate 320. In this case, the concave portion 325 may be provided on the front end portions of the installation grooves 811 and 831.

When the dust storage part 300 is disposed by being inserted into the correct location of the dust storage housing part 800, the convex portions 819 and 839 and the concave portion 325 may be coupled. The coupling of the convex portions 819 and 839 and the concave portion 325 may be recognized by the touch of the user who holds the dust bag handle 321.

As described above, when the dust storage part 300 is disposed at the correct location, it means that the axis a1 of the flow path connection member 870 and the center axis a2 of the dust hole 311 of the dust bag 310 (or the dust hole 328 of the upper plate 320) are coaxial with each other. When the dust storage part 300 is not located at the correct location, the suctioned dust may leak to a gap between the flow path connection member 870 and the dust hole 328 of the upper plate 320. That is, complete sealing is not achieved, and dust may fly, thereby contaminating the inside of the cleaner station 10.

As in the embodiment of the present invention, when the dust storage part 300 is disposed at the correct location and the correct location installation detection structure of the coupled convex portions 819 and 839 and the concave portion 325 is used, there is an advantage that the user can recognize the state in which the dust storage part 300 is not completely sealed with the suction flow path 700 through touch. In addition, this prevents the inside of the cleaner station 10 from being contaminated with flying dust.

Although the specific embodiment of the present invention has been described and shown above, the present invention is not limited to the described embodiments. Therefore, the scope of the present invention should not be determined by the described embodiments but should be determined by the claims.

## Claims

1. A cleaner station (10) connected to a dust bin (2160) of a cleaner (20) to suction dust from the dust bin (2160), the cleaner station (10) comprising:
a dust storage part (300) configured to store dust suctioned from the cleaner (20);
a dust storage housing part (800) in which an accommodation space having the dust storage part (300) accommodated therein is formed and in which the dust storage part (300) is detachably provided; and
a housing cover part (900) configured to open and close a partial area of the dust storage housing part (800),
**characterized in that** the dust storage housing part (800) includes:
a fixed holder (810) fixedly disposed to an upper surface of the accommodation space and including an installation groove (811, 831) formed so that the dust storage part (300) is formed to slide; and
a stopper (850) connected to the fixed holder (810) to linearly reciprocate a predetermined distance,
wherein the stopper (850) is configured to linearly move in a direction that interferes with the housing cover part (900) when the dust storage part (300) is separated from the installation groove (811, 831).

2. The cleaner station (10) of claim 1, wherein the stopper (850) is configured to:
be linearly moved to a first location by being pushed while being supported by a partial area of the dust storage part (300) when the dust storage part (300) slides in a direction that is inserted into the installation groove (811, 831); and
linearly move to a second location interfering with a partial area of the housing cover part (900) as the support by the dust storage part (300) is released when the dust storage part (300) slides in a direction that is separated from the installation groove (811, 831).

3. The cleaner station (10) of claim 2, wherein the linear movement from the first location to the second location is a downward movement by gravity.

4. The cleaner station (10) of claim 2, wherein a spring configured to apply an elastic restoring force to the second location is coupled to the stopper (850), and
the linear movement from the first location to the second location is a movement by the elastic restoring force of the spring.

5. The cleaner station (10) of claim 1, wherein the fixed holder (810) further includes a guide rail (817) configured to guide the stopper (850) to linearly move in a vertical direction.

6. The cleaner station (10) of claim 5, wherein the stopper (850) includes:
a rail insertion groove (857) into which the guide rail (817) is inserted; and
a hook (859) protruding and extending from a rear end of the rail insertion groove (857) to a center of the stopper (850) and coupled by being caught on the guide rail (817).

7. The cleaner station (10) of claim 1, wherein the fixed holder (810) further includes:
a rail groove (815) formed from a front surface to a rear and providing a space in which the stopper (850) moves in a vertical direction; and
a support surface (815a) supporting a partial surface of the stopper (850) at a lower end portion of the rail groove (815) to stop the downward movement.

8. The cleaner station (10) of claim 1, wherein the fixed holder (810) further includes:
a first installation surface (813, 833) disposed on a path along which the dust storage part (300) slides and extending in a horizontal direction; and
a second installation surface (814, 834) connected to a rear end of the first installation surface (813, 833) and extending to have a rearward and upward inclination.

9. The cleaner station (10) of claim 1, wherein the housing cover part (900) includes an interference member (930) protruding toward an inside of the dust storage housing part (800), and
the interference member (930) is disposed at a location at which closing of the dust storage housing part (800) is interfered by the stopper (850) in a state in which the dust storage part (300) has been separated from the installation groove (811, 831).

10. The cleaner station (10) of claim 1, wherein the dust storage part (300) includes:
a dust bag (310) configured to accommodate and store dust suctioned from the cleaner (20) therein; and
an upper plate (320) provided to be coupled to an external upper surface of the dust bag (310) and inserted into the installation groove (811, 831) in a sliding manner, and
the upper plate (320) includes:
a dust bag handle (321) provided at a front of the dust storage part (300) and gripped by a user; and
an inclined rib (323) formed to protrude and extend rearward from both left and right end portions of the dust bag handle (321) and formed to extend to have a rearward and upward inclination.

11. The cleaner station (10) of claim 10, wherein the stopper (850) includes
a guide inclined surface (852) having a downward inclination in an insertion direction of the upper plate (320), and when the upper plate is inserted into the installation groove (811, 831), the stopper (850) moves upward as the upper plate (320) slides toward an inside of the accommodation space along the guide inclined surface (852).

12. The cleaner station (10) of claim 10, wherein the dust storage part (300) further includes
a lower plate (324) coupled to an inner upper surface of the dust bag (310) and provided with a transmissive panel (327) to transmit sterilization light.

13. The cleaner station (10) of claim 1, further comprising a micro switch in contact with the dust storage part (300) to detect whether the dust storage part (300) has been disposed at a correct location when the dust storage part (300) is inserted into the dust storage housing part (800).

14. A cleaner station (10) connected to a dust bin (2160) of a cleaner (20) to suction dust from the dust bin (2160),
the cleaner station (10) comprising:
a dust storage part (300) configured to store dust suctioned from the cleaner (20);
a dust storage housing part (800) in which an accommodation space having the dust storage part (300) accommodated therein is formed and in which the dust storage part (300) is detachably provided; and
a housing cover part (900) configured to open and close a partial area of the dust storage housing part (800),
**characterized in that** the dust storage housing part (800) includes:
a fixed holder (810) fixedly disposed to an upper surface of the accommodation space and including an installation groove (811, 831) formed so that the dust storage part (300) is formed to slide, one sides of the installation groove (811, 831) and the dust storage part (300) are each provided with a convex portion (819, 839) and the other sides are each provided with a concave portion (325) having a shape corresponding to the convex portion (819, 839), and
when the dust storage part (300) is disposed by being inserted at a correction location of the dust storage housing part (800), the convex portion (819, 839) and the concave portion (325) are coupled.

15. The cleaner station (10) of claim 14, wherein the dust storage part (300) includes:
a dust bag (310) configured to accommodate and store dust suctioned from the cleaner (10) therein; and
an upper plate (320) provided to be coupled to an external upper surface of the dust bag (310) and inserted into the installation groove (811, 831) in a sliding manner, and
the convex portion (819, 839) or the concave portion (325) is provided on one end portion of a sliding movement path of the upper plate (320).

16. The cleaner station (10) of claim 14, further comprising a stopper (850) linearly moves in a direction that interferes with the housing cover part (900) when the dust storage part (300) is separated from the installation groove (811, 831),
wherein the stopper (850) is configured to:
be moved upward by being pushed by a partial area of the dust storage part (300) when the dust storage part (300) slides in a direction that is inserted into the installation groove (811, 831); and be moved downward by gravity to a location interfering with a partial area of the housing cover part (900) as the support by the dust storage part (300) is released when the dust storage part (300) slides in a direction that is separated from the installation groove (811, 831).

## Patentansprüche

1. Reinigerstation (10), die mit einem Staubbehälter (2160) einer Reinigungsvorrichtung (20) verbunden ist, um Staub aus dem Staubbehälter (2160) zu saugen, wobei die Reinigerstation (10) Folgendes umfasst:
eine Staubaufbewahrungskomponente (300), die konfiguriert ist, Staub, der von der Reinigungsvorrichtung (20) eingesaugt worden ist, aufzubewahren;
eine Staubaufbewahrungs-Gehäusekomponente (800), in der ein Aufnahmeraum, in dem die Staubaufbewahrungskomponente (300) aufgenommen ist, gebildet ist und in der die Staubaufbewahrungskomponente (300) entnehmbar vorgesehen ist; und
eine Gehäuseabdeckkomponente (900), die konfiguriert ist, einen Teilbereich der Staubaufbewahrungs-Gehäusekomponente (800) zu öffnen und zu verschließen,
**dadurch gekennzeichnet, dass** die Staubaufbewahrungs-Gehäusekomponente (800) Folgendes enthält:
einen feststehenden Halter (810), der an einer oberen Fläche des Aufnahmeraums fest angeordnet ist und eine Installationsnut (811, 831) aufweist, die derart gebildet ist, dass die Staubaufbewahrungskomponente (300) auf eine Weise ausgebildet ist, dass sie gleitet; und
ein Anschlagelement (850), das mit dem feststehenden Halter (810) verbunden ist, derart, dass es sich um einen vorgegebenen Abstand geradlinig hin und her bewegt,
wobei das Anschlagelement (850) konfiguriert ist, sich in einer Richtung geradlinig zu bewegen, in der es auf die Gehäuseabdeckkomponente (900) einwirkt, wenn die Staubaufbewahrungskomponente (300) von der Installationsnut (811, 831) getrennt wird.

2. Reinigerstation (10) nach Anspruch 1, wobei das Anschlagelement (850) konfiguriert ist:
geradlinig an einen ersten Ort bewegt zu werden, indem es geschoben wird, während es durch einen Teilbereich der Staubaufbewahrungskomponente (300) getragen wird, wenn die Staubaufbewahrungskomponente (300) in einer Richtung gleitet, in der sie in die Installationsnut (811, 831) eingeführt wird; und
sich geradlinig an einen zweiten Ort zu bewegen, an dem es auf einen Teilbereich der Gehäuseabdeckkomponente (900) einwirkt, wenn die Unterstützung durch die Staubaufbewahrungskomponente (300) freigegeben wird, wenn die Staubaufbewahrungskomponente (300) in einer Richtung gleitet, in der sie von der Installationsnut (811, 831) getrennt wird.

3. Reinigerstation (10) nach Anspruch 2, wobei die geradlinige Bewegung vom ersten Ort an den zweiten Ort eine schwerkraftbedingte Bewegung nach unten ist.

4. Reinigerstation (10) nach Anspruch 2, wobei eine Feder, die konfiguriert ist, eine elastische Rückstellkraft auf den zweiten Ort auszuüben, mit dem Anschlagelement (850) gekoppelt ist, und
die geradlinige Bewegung vom ersten Ort an den zweiten Ort eine Bewegung durch die elastische Rückstellkraft der Feder ist.

5. Reinigerstation (10) nach Anspruch 1, wobei der feststehende Halter (810) ferner eine Führungsschiene (817) enthält, die konfiguriert ist, das Anschlagelement (850) derart zu führen, dass es sich geradlinig in einer vertikalen Richtung bewegt.

6. Reinigerstation (10) nach Anspruch 5, wobei das Anschlagelement (850) Folgendes aufweist:
eine Schieneneinführungsnut (857), in die die Führungsschiene (817) eingeführt wird; und
einen Haken (859), der von einem hinteren Ende der Schieneneinführungsnut (857) vorsteht und sich zu einer Mitte des Anschlagelements (850) erstreckt und gekoppelt wird, indem er auf der Führungsschiene (817) eingefangen wird.

7. Reinigerstation (10) nach Anspruch 1, wobei der feststehende Halter (810) ferner Folgendes aufweist:
eine Schienennut (815), die von einer vorderen Fläche bis zu einer Rückseite gebildet ist und einen Raum bereitstellt, in dem sich das Anschlagelement (850) in einer vertikalen Richtung bewegt; und
eine Trägerfläche (815a), die eine Teilfläche des Anschlagelements (850) an einem unteren Endabschnitt der Schienennut (815) trägt, um die Bewegung nach unten anzuhalten.

8. Reinigerstation (10) nach Anspruch 1, wobei der feststehende Halter (810) ferner Folgendes enthält:
eine erste Installationsfläche (813, 833), die auf einem Weg angeordnet ist, entlang dessen die Staubaufbewahrungskomponente (300) gleitet, und sich in einer horizontalen Richtung erstreckt; und
eine zweite Installationsfläche (814, 834), die mit einem hinteren Ende der ersten Installationsfläche (813, 833) verbunden ist und sich derart erstreckt, das sie eine Neigung nach hinten und nach oben aufweist.

9. Reinigerstation (10) nach Anspruch 1, wobei die Gehäuseabdeckkomponente (900) ein Eingriffelement (930) enthält, das zu einer Innenseite der Staubaufbewahrungs-Gehäusekomponente (800) vorsteht, und
das Eingriffelement (930) an einem Ort angeordnet ist, an dem in einem Zustand, in dem die Staubaufbewahrungskomponente (300) von der Installationsnut (811, 831) getrennt worden ist, durch das Anschlagelement (850) in das Schließen der Staubaufbewahrungs-Gehäusekomponente (800) eingegriffen wird.

10. Reinigerstation (10) nach Anspruch 1, wobei die Staubaufbewahrungskomponente (300) Folgendes enthält:
einen Staubbeutel (310), der konfiguriert ist, Staub, der von der Reinigungsvorrichtung (20) eingesaugt worden ist, darin aufzunehmen und aufzubewahren; und
eine obere Platte (320), die derart vorgesehen ist, dass sie mit einer äußeren oberen Fläche des Staubbeutels (310) gekoppelt ist und auf eine gleitende Weise in die Installationsnut (811, 831) eingeführt wird, und
wobei die obere Platte (320) Folgendes enthält:
einen Staubbeutelgriff (321), der an einer Vorderseite der Staubaufbewahrungskomponente (300) vorgesehen ist und von einen Benutzer ergriffen wird; und
eine geneigte Rippe (323), die derart gebildet ist, dass sie sowohl vom linken als auch vom rechten Endabschnitt des Staubbeutelgriffs (321) vorsteht und sich nach hinten erstreckt, und derart gebildet ist, dass sie sich so erstreckt, dass sie eine Neigung nach hinten und nach oben aufweist.

11. Reinigerstation (10) nach Anspruch 10, wobei das Anschlagelement (850) Folgendes enthält:
eine geneigte Führungsfläche (852), die eine Neigung nach unten in einer Einführungsrichtung der oberen Platte (320) aufweist, und wobei dann, wenn die obere Platte in die Installationsnut (811, 831) eingeführt wird, das Anschlagelement (850) sich nach oben bewegt, während die obere Platte (320) in Richtung einer Innenseite des Aufnahmeraums entlang der geneigten Führungsfläche (852) gleitet.

12. Reinigerstation (10) nach Anspruch 10, wobei die Staubaufbewahrungskomponente (300) ferner Folgendes enthält:
eine untere Platte (324), die mit einer oberen Innenfläche des Staubbeutels (310) gekoppelt ist und mit einer lichtdurchlässigen Platte (327), um ein Sterilisationslicht zu übertragen, versehen ist.

13. Reinigerstation (10) nach Anspruch 1, die ferner einen Mikroschalter in Kontakt mit der Staubaufbewahrungskomponente (300) umfasst, um zu detektieren, ob die Staubaufbewahrungskomponente (300) an einem richtigen Ort angeordnet worden ist, wenn die Staubaufbewahrungskomponente (300) in die Staubaufbewahrungs-Gehäusekomponente (800) eingeführt wird.

14. Reinigerstation (10), die mit einem Staubbehälter (2160) einer Reinigungsvorrichtung (20) verbunden ist, um Staub aus dem Staubbehälter (2160) zu saugen,
wobei die Reinigerstation (10) Folgendes umfasst:
eine Staubaufbewahrungskomponente (300), die konfiguriert ist, Staub, der von der Reinigungsvorrichtung (20) eingesaugt worden ist, aufzubewahren;
eine Staubaufbewahrungs-Gehäusekomponente (800), in der ein Aufnahmeraum, in dem die Staubaufbewahrungskomponente (300) aufgenommen ist, gebildet ist und in der die Staubaufbewahrungskomponente (300) entnehmbar vorgesehen ist; und
eine Gehäuseabdeckkomponente (900), die konfiguriert ist, einen Teilbereich der Staubaufbewahrungs-Gehäusekomponente (800) zu öffnen und zu verschließen,
**dadurch gekennzeichnet, dass** die Staubaufbewahrungs-Gehäusekomponente (800) Folgendes enthält:
einen feststehenden Halter (810), der an einer oberen Fläche des Aufnahmeraums fest angeordnet ist und eine Installationsnut (811, 831) aufweist, die derart gebildet ist, dass die Staubaufbewahrungskomponente (300) auf eine Weise ausgebildet ist, dass sie gleitet, wobei je eine Seite der Installationsnut (811, 831) und der Staubaufbewahrungskomponente (300) jeweils mit einem konvexen Abschnitt (819, 839) versehen sind und die anderen Seiten jeweils mit einem konkaven Abschnitt (325) versehen sind, der eine Form aufweist, die dem konvexen Abschnitt (819, 839) entspricht, und
dann, wenn die Staubaufbewahrungskomponente (300) angeordnet wird, indem sie an einen richtigen Ort der Staubaufbewahrungs-Gehäusekomponente (800) eingeführt wird, der konvexe Abschnitt (819, 839) und der konkave Abschnitt (325) gekoppelt werden.

15. Reinigerstation (10) nach Anspruch 14, wobei die Staubaufbewahrungskomponente (300) Folgendes enthält:
einen Staubbeutel (310), der konfiguriert ist, Staub, der von der Reinigungsvorrichtung (10) eingesaugt worden ist, darin aufzunehmen und aufzubewahren; und
eine obere Platte (320), die derart vorgesehen ist, das sie mit einer äußeren oberen Fläche des Staubbeutels (310) gekoppelt ist und auf eine gleitende Weise (811, 831) in die Installationsnut (811, 831) eingeführt wird, und
wobei der konvexe Abschnitt (819, 839) oder der konkave Abschnitt (325) auf einem Endabschnitt eines Gleitbewegungsweges der oberen Platte (320) vorgesehen ist.

16. Reinigerstation (10) nach Anspruch 14, die ferner ein Anschlagelement (850) umfasst, das sich in einer Richtung geradlinig bewegt, in der es auf die Gehäuseabdeckkomponente (900) einwirkt, wenn die Staubaufbewahrungskomponente (300) von der Installationsnut (811, 831) getrennt wird,
wobei das Anschlagelement (850) konfiguriert ist:
nach oben bewegt zu werden, indem es durch einen Teilbereich der Staubaufbewahrungskomponente (300) geschoben wird, wenn die Staubaufbewahrungskomponente (300) in einer Richtung gleitet, in der sie in die Installationsnut (811, 831) eingeführt wird; und durch Schwerkraft nach unten an einen Ort bewegt zu werden, an dem es auf einen Teilbereich des Gehäuseabdeckkomponente (900) einwirkt, wenn die Unterstützung durch die Staubaufbewahrungskomponente (300) freigegeben wird, wenn die Staubaufbewahrungskomponente (300) in einer Richtung gleitet, in der sie von der Installationsnut (811, 831) getrennt wird.

## Revendications

1. Station d'appareil de nettoyage (10) reliée à un bac à poussière (2160) d'un dispositif de nettoyage (20) pour aspirer de la poussière à partir du bac à poussière (2160), la station de dispositif de nettoyage (10) comportant :
une partie de stockage de poussière (300) configurée pour stocker la poussière aspirée à partir du dispositif de nettoyage (20) ;
une partie de boîtier de stockage de poussière (800) dans laquelle est formé un espace de réception ayant la partie de stockage de poussière (300) reçue dans celui-ci et dans laquelle la partie de stockage de poussière (300) est fournie de manière détachable ; et
une partie de couvercle de boîtier (900) configurée pour ouvrir et fermer une zone partielle de la partie de boîtier de stockage de poussière (800),
**caractérisé en ce que** la partie de boîtier de stockage de poussière (800) inclut :
un support fixe (810) disposé fixement sur une surface supérieure de l'espace de réception et incluant une rainure d'installation (811, 831) formée de sorte que la partie de stockage de poussière (300) est formée de manière à coulisser ; et
un bouchon (850) relié au support fixe (810) pour être linéairement animé d'un mouvement de va-et-vient sur une distance prédéterminée,
dans laquelle le bouchon (850) est configuré pour se déplacer linéairement dans une direction qui interfère avec la partie de couvercle de boîtier (900) lorsque la partie de stockage de poussière (300) est séparée de la rainure d'installation (811, 831).

2. Station de dispositif de nettoyage (10) selon la revendication 1, dans laquelle le bouchon (850) est configuré pour :
être déplacé linéairement jusqu'à un premier emplacement en étant poussé tout en étant supporté par une zone partielle de la partie de stockage de poussière (300) lorsque la partie de stockage de poussière (300) coulisse dans une direction qui est insérée dans la rainure d'installation (811, 831) ; et
se déplacer linéairement jusqu'à un second emplacement interférant avec une zone partielle de la partie couvercle de boîtier (900) dès que le support par la partie de stockage de poussière (300) est libéré lorsque la partie de stockage de poussière (300) coulisse dans une direction qui est séparée de la rainure d'installation (811, 831).

3. Station de dispositif de nettoyage (10) selon la revendication 2, dans laquelle le mouvement linéaire du premier emplacement au second emplacement est un mouvement vers le bas, par gravité.

4. Station de dispositif de nettoyage (10) selon la revendication 2, dans laquelle un ressort configuré pour appliquer une force de rappel élastique au second emplacement est couplé au bouchon (850), et
le mouvement linéaire du premier emplacement au second emplacement est un mouvement provoqué par la force de rappel élastique du ressort.

5. Station de dispositif de nettoyage (10) selon la revendication 1, dans laquelle le support fixe (810) inclut en outre un rail de guidage (817) configuré pour guider le bouchon (850) pour qu'il se déplace linéairement dans une direction verticale.

6. Station de dispositif de nettoyage (10) selon la revendication 5, dans laquelle le bouchon (850) inclut :
une rainure d'insertion de rail (857) dans laquelle le rail de guidage (817) est inséré ; et
un crochet (859) faisant saillie et s'étendant à partir d'une extrémité arrière de la rainure d'insertion de rail (857) jusqu'à un centre du bouchon (850) et couplé en étant attrapé sur le rail de guidage (817).

7. Station de dispositif de nettoyage (10) selon la revendication 1, dans laquelle le support fixe (810) inclut en outre :
une rainure de rail (815) formée à partir d'une surface avant jusqu'à un arrière et fournissant un espace dans lequel le bouchon (850) se déplace dans une direction verticale ; et
une surface de support (815a) supportant une surface partielle du bouchon (850) sur une portion d'extrémité inférieure de la rainure de rail (815) pour arrêter le mouvement vers le bas.

8. Station de dispositif de nettoyage (10) selon la revendication 1, dans laquelle le support fixe (810) inclut en outre :
une première surface d'installation (813, 833) disposée sur un chemin le long duquel la partie de stockage de poussière (300) coulisse et s'étendant dans une direction horizontale ; et
une seconde surface d'installation (814, 834) reliée à une extrémité arrière de la première surface d'installation (813, 833) et s'étendant de manière à avoir une inclinaison vers l'arrière et vers le haut.

9. Station de dispositif de nettoyage (10) selon la revendication 1, dans laquelle la partie de couvercle de boîtier (900) inclut un élément d'interférence (930) faisant saillie vers un intérieur de la partie de boîtier de stockage de poussière (800), et
l'élément d'interférence (930) est disposé à un emplacement au niveau duquel une fermeture de la partie de boîtier de stockage de poussière (800) est perturbée par le bouchon (850) dans un état dans lequel la partie de stockage de poussière (300) a été séparée de la rainure d'installation (811, 831).

10. Station de dispositif de nettoyage (10) selon la revendication 1, dans laquelle la partie de stockage de poussière (300) inclut :
un sac à poussière (310) configuré pour recevoir et stocker de la poussière aspirée par le dispositif de nettoyage (20) dans celui-ci ; et
une plaque supérieure (320) agencée de manière à être couplée à une surface supérieure externe du sac à poussière (310) et insérée dans la rainure d'installation (811, 831) d'une manière coulissante, et
la plaque supérieure (320) inclut :
une poignée de sac à poussière (321) agencée sur un avant de la partie de stockage de poussière (300) et saisie par un utilisateur ; et
une nervure inclinée (323) formée pour faire saillie et s'étendre vers l'arrière à partir des deux portions d'extrémité gauche et droite de la poignée de sac à poussière (321) et formée pour s'étendre de manière à avoir une inclinaison vers l'arrière et vers le haut.

11. Station de dispositif de nettoyage (10) selon la revendication 10, dans laquelle le bouchon (850) inclut :
une surface inclinée de guidage (852) ayant une inclinaison vers le bas dans une direction d'insertion de la plaque supérieure (320), et lorsque la plaque supérieure est insérée dans la rainure d'installation (811, 831), le bouchon (850) se déplace vers le haut dès que la plaque supérieure (320) coulisse vers un intérieur de l'espace de réception le long de la surface inclinée de guidage (852).

12. Station de dispositif de nettoyage (10) selon la revendication 10, dans laquelle la partie de stockage de poussière (300) inclut en outre :
une plaque inférieure (324) couplée à une surface supérieure intérieure du sac à poussière (310) et pourvue d'un panneau transmissif (327) pour transmettre de la lumière de stérilisation.

13. Station de dispositif de nettoyage (10) selon la revendication 1, comportant en outre un micro-commutateur en contact avec la partie de stockage de poussière (300) pour détecter si la partie de stockage de poussière (300) a été disposée à un emplacement correct lorsque la partie de stockage de poussière (300) est insérée dans la partie de boîtier de stockage de poussière (800).

14. Station de dispositif de nettoyage (10) reliée à un bac à poussière (2160) d'un dispositif de nettoyage (20) pour aspirer la poussière du bac à poussière (2160),
la station de dispositif de nettoyage (10) comprenant :
une partie de stockage de poussière (300) configurée pour stocker la poussière aspirée à partir du dispositif de nettoyage (20) ;
une partie de boîtier de stockage de poussière (800) dans laquelle est formé un espace de réception ayant la partie de stockage de poussière (300) reçue dans celui-ci et dans laquelle la partie de stockage de poussière (300) est fournie de manière détachable ; et
une partie de couvercle de boîtier (900) configurée pour ouvrir et fermer une zone partielle de la partie de boîtier de stockage de poussière (800),
**caractérisé en ce que** la partie de boîtier de stockage de poussière (800) inclut :
un support fixe (810) disposé fixement sur une surface supérieure de l'espace de réception et incluant une rainure d'installation (811, 831) formée de sorte que la partie de stockage de poussière (300) est formée pour coulisser, des premiers côtés de la rainure d'installation (811, 831) et de la partie de stockage de poussière (300) sont chacun pourvus d'une portion convexe (819, 839) et les seconds côtés sont chacun pourvus d'une portion concave (325) ayant une forme correspondant à la portion convexe (819), et
lorsque la partie de stockage de poussière (300) est disposée en étant insérée à un emplacement de correction de la partie de boîtier de stockage de poussière (800), la portion convexe (819, 839) et la portion concave (325) sont couplées.

15. Station de dispositif de nettoyage (10) selon la revendication 14, dans laquelle la partie de stockage de poussière (300) inclut :
un sac à poussière (310) configuré pour recevoir et stocker de la poussière aspirée par le dispositif de nettoyage (10) dans celui-ci ; et
une plaque supérieure (320) agencée de manière à être couplée à une surface supérieure externe du sac à poussière (310) et insérée dans la rainure d'installation (811, 831) d'une manière coulissante, et
la portion convexe (819, 839) ou la portion concave (325) est agencée sur une portion d'extrémité d'un trajet de mouvement coulissant de la plaque supérieure (320).

16. Station de dispositif de nettoyage (10) selon la revendication 14, comportant en outre un bouchon (850) qui se déplace linéairement dans une direction qui interfère avec la partie de couvercle de boîtier (900) lorsque la partie de stockage de poussière (300) est séparée de la rainure d'installation (811, 831),
dans laquelle le bouchon (850) est configuré pour :
être déplacé vers le haut en étant poussé par une zone partielle de la partie de stockage de poussière (300) lorsque la partie de stockage de poussière (300) coulisse dans une direction qui est insérée dans la rainure de montage (811, 831) ; et être déplacé vers le bas, par gravité, jusqu'à un emplacement interférant avec une zone partielle de la partie de couvercle de boîtier (900) dès que le support par la partie de stockage de poussière (300) est libéré lorsque la partie de stockage de poussière (300) coulisse dans une direction qui est séparée de la rainure d'installation (811, 831).
